# EUROPEAN PATENT APPLICATION

(11) **EP 4 545 966 A1**
(43) Date of publication of application: **30.04.2025**
(21) Application number: 22947595.9
(22) Date of filing: 23.08.2022
(51) Int. Cl.: G01N 33/541, G01N 33/58, G01N 21/64

(54) **REAGENT COMBINATION, KIT, DETECTION SYSTEM AND DETECTION METHOD FOR DETECTING SMALL MOLECULAR SUBSTANCE**

(30) Priority: 23.06.2022 CN 202210718352
(71) Applicant: Nanjing Poclight Biotechnology Co., Ltd, Nanjing, Jiangsu 210061 (CN)
(72) Inventor: CAO, Dan, Nanjing, Jiangsu 210061 (CN); CHENG, Shun, Nanjing, Jiangsu 210061 (CN)
(74) Representative: Murgitroyd & Company
(86) International application number: PCT/CN2022/114296
(87) International publication number: WO 2023/245854

(57) **Abstract**

A reagent combination, a kit, a detection system and a detection method for detecting a small molecule substance. **In** the present application, in the condition that a solution to be tested does not contain or contains a small amount of the small molecule substance, an antibody binds to a substrate protein conjugate to form an immune complex, a stem-loop structure is generated by means of a complementary pairing among a plurality of nucleic acid molecules, a first fluorescent group excites a second fluorescent group to emit a second fluorescence on the basis of fluorescence resonance energy transfer, and the content of the small molecule substance is calculated. **In** the condition that the solution to be tested contains a large amount of the small molecule substance, the immune complex and the stem-loop structure cannot be formed, and the second fluorescent group cannot emit the second fluorescent, so that it is determined that the solution to be tested contains a large amount of the small molecule substances. The detection method of the present application is simple, has low background interference, high sensitivity and small measurement error.

## Description

### CROSS-REFERENCE TO THE RELATED APPLICATION

The present application claims priority to Chinese Patent Application No. 202210718352. 5, filed on June 23, 2022 with China Patent Office, entitled with "REAGENT COMBINATION, KIT, DETECTION SYSTEM AND DETECTION METHOD FOR DETECTING SMALL MOLECULE SUBSTANCE", the entire contents of which are incorporated herein by reference.

### TECHNICAL FIELD

The present application belongs to the field of chemiluminescence detection technology, and specifically relates to a reagent combination, a kit, a detection system and a detection method for detecting a small molecule substance.

### BACKGROUND

A human blood is a complex multi-component system containing small molecules substance such as a variety of regulatory factors and hormones. Among them, some small molecule substances are not secreted in a healthy tissue, but are secreted in a diseased tissue. These abnormally secreted small molecule substances will enter the blood. Therefore, whether there is the healthy tissue or the diseased tissue in the human body may be judged on a basis of the presence or absence of such small molecule substances in the blood. A chemiluminescence immunoassay (CLIA) may be used to detect the absence or presence of the small molecule substances in blood. Such method connects a luminescent group to an antibody against a small molecule substance to make a detection reagent. When there are no small molecule substances in the blood sample, the antibody cannot specifically bind to the small molecule substance in the blood. At this time, the luminescent group generally does not have luminescent activity and will not produce strong fluorescence strength in addition to the background fluorescence. When there is a small molecule substance in the solution to be tested, the antibody can specifically bind to the small molecule substance, and the luminescent group emits fluorescence under the action of the luminescent substrate. Therefore, the presence or absence of the small molecule substance in the blood may be determined by using a chemiluminescence detection instrument to detect the presence or absence of fluorescence in the solution to be tested.

However, the above method uses a single fluorescent group to emit the fluorescence, and determines whether there are the small molecule substances on a basis of whether the single fluorescent group emits the fluorescence. Since the single fluorescent group will emit the background fluorescence in the absence of the small molecule substance, this background fluorescence will affect the acquisition of fluorescence, thus causing a large error when measuring the content of the small molecule substance.

### SUMMARY

The purpose of some embodiments of the present application is to provide a reagent combination, a kit, a detection system and a detection method for detecting the small molecule substance, which can avoid the impact of the background fluorescence of the single fluorescent group of the existing chemiluminescence detection method on the detection results. Some of the embodiments mentioned herein may be derived from the same embodiment or from different embodiments.

In order to solve the above technical problem, some embodiments of the present application provide a reagent combination for detecting a small molecule substance. The reagent combination includes at least: a first conjugate, a second conjugate, a third conjugate and an oxidation inhibiting reagent.

The first conjugate is formed at least by coupling a first nucleic acid molecule to an antibody. The first nucleic acid molecule contains a first hybridization zone and a second hybridization zone. The first hybridization zone and the second hybridization zone are not directly adjacent, but are separated by 2 or 5 nucleotides. The complementarity determining region (CDR) of the antibody can specifically bind to the small molecule substance. In various embodiments of the present application, the nucleic acid molecules each are single-stranded DNA. In the present application, when describing each single-stranded DNA, the connection manner thereof is from the 5' end to the 3' end from left to right.

The second conjugate is formed at least by sequentially coupling a substrate protein conjugate, a second nucleic acid molecule and a second fluorescent group. The substrate protein conjugate is formed at least by coupling a small molecule substrate to a scaffold protein. The small molecule substrate is the same substance as the small molecule substance to be measured. For the sake of distinction, the substance bound to the scaffold protein is called as the small molecule substrate, and the substance free in the solution to be tested is called as the small molecule substance. The second nucleic acid molecule has a third hybridization zone and a fourth hybridization zone. The third hybridization zone is directly connected to the fourth hybridization zone, with no intervening nucleotides therebetween. The third hybridization zone is complementary to the second hybridization zone. For the antibody, the small molecule substrate has only one antigenic epitope, which can specifically bind to the CDR of the antibody, and the binding is reversible and can be reversed by the small molecule substance free in the solution to be tested. That is, the small molecule substance in the free state and the small molecule substrate in the bound state can competitively bind to the antigenic epitope of the antibody. The CDR of each antibody can only bind to one small molecule substrate or one small molecule substance at the same time.

The third conjugate is formed at least by coupling a first fluorescent group and a third nucleic acid molecule. The third nucleic acid molecule contains a fifth hybridization zone and a sixth hybridization zone. The fifth hybridization zone is directly connected to the sixth hybridization zone, with no intervening nucleotides therebetween. The fifth hybridization zone is complementary to the fourth hybridization zone, and the sixth hybridization zone is complementary to the first hybridization zone. The first nucleic acid molecule, the second nucleic acid molecule and the third nucleic acid molecule are all the single-stranded DNA. By the complementary pairing of the above single-stranded DNA molecules, the first nucleic acid molecule, the second nucleic acid molecule and the third nucleic acid molecule can be assembled into a Stem-Loop structure. The structure of the stem is ⊥-shaped, and the regions paired with each other present a double helix structure.

In some embodiments, in a case that the antibody binds to the small molecule substrate in the substrate protein conjugate rather than the free small molecule substance in the solution to be tested, the first fluorescent group emits a first fluorescence at the condition that it can be oxidized by the oxidant and in the absence of the anti-oxidant, the first fluorescence excites the second fluorescent group to emit the second fluorescence as an excitation light to excite on a basis of the fluorescence resonance energy transfer effect in the condition that the first nucleic acid molecule, the second nucleic acid molecule and the third nucleic acid molecule are paired with each other in order to obtain the content of the small molecule substance on a basis of the intensity of the second fluorescence. If the intensity of the second fluorescence is not obtained at all, it means that the content of the small molecule substance is too high, and all the antibodies are bound to the free small molecule substance, rather than to the small molecule substrate in the substrate protein conjugate, exceeding the detection limit. At this time, the content of the small molecule substance cannot be obtained on a basis of the intensity of the fluorescence.

In some embodiments, when the concentration of the small molecule substance in the solution to be tested is low, the binding degree of the small molecule substance to the antibody is weak, and the binding degree of the small molecule substrate to the antibody is strong, then the intensity of the second fluorescence is stronger. powerful. Therefore, the concentration of the small molecule substance in the solution to be tested is inversely proportional to the intensity of the second fluorescence. The functional relationship between the intensity of the second fluorescence and the content of the small molecule substance may be obtained in advance, thereby establishing a numerical relationship in the one-to-one correspondence between the two. Therefore, the measurement of the content of the small molecule substance may be converted into the numerical measurement of the intensity of the fluorescence.

The oxidation inhibiting agent includes an antioxidant. The antioxidant can inhibit the first fluorescent group from being oxidized to emit the first fluorescence. The first fluorescent group in various embodiments of the present application are an oxidative luminescence, rather than emitting a fluorescent light when exposed to the excitation light. Therefore, it is necessary to minimize the oxidation of the first fluorescent group by the oxidizing substance in the solution to be tested to generate a background fluorescence. The background fluorescence is a type of noise, which will affect the true value of the fluorescence measurement, thereby affecting the accuracy of the measurement result of the small molecule content. The antioxidant and the oxidant cannot exist in the sample to be tested at the same time, so as to prevent the oxidative luminescence effect of the oxidant on the first fluorescent group from being neutralized by the antioxidant. Therefore, the antioxidant need to be removed before adding the oxidant.

In some embodiments of the present application, the following basic conditions are required for the fluorescence resonance energy transfer effect between the first fluorescent group and the second fluorescent group: the first nucleic acid molecule, the second nucleic acid molecule, and the third nucleic acid molecule are completely complementarily paired in the condition that there is no small molecule substance in the solution to be tested. At this time, the intensity of the generated second fluorescence is the largest. The first nucleic acid molecule, the second nucleic acid molecule, and the third nucleic acid molecule are partially complementarily paired in the condition that there is a lower concentration of the small molecule substance in the solution to be tested. At this time, the second fluorescence will also be generated. The complementary pairing will form a stem-loop structure such that the spacing distance between the first fluorescent group and the second fluorescent group is smaller than the limit spacing distance at which fluorescence resonance energy transfer can occur, example, in the range of 70 angstroms to 99 angstroms, or in the range of 7 nm to 10 nm.

In some embodiments of the present application, the first fluorescent group may be an acridinium ester, and the second fluorescent group may be a quantum dot.

In some embodiments of the present application, the maximum emission wavelength of the first fluorescent group is 430 nm.

The maximum absorption wavelength of the second fluorescent group may be in the range of 420 nm to 520 nm, for example, it may be 470 nm. The maximum emission wavelength of the second fluorescent group may be in the range of 595 nm to 615 nm, for example, it may be 605 nm. In some embodiments of the present application, the quantum dot is a core-shell structure quantum dot, the core layer material of which is selected from one or more of CdSe, CdS, CdTe, CdSeTe, CdZnS, ZnTe, CdSeS, PbS and PbTe, and the shell material of which is selected from one or more of ZnS, ZnSe, ZnSeS, PbS and PbSeS.

In some embodiments of the present application, the particle size range of the quantum dot may be 3 nm to 5 nm, or may be 4.1 nm to 4.2 nm.

In some embodiments of the present application, the sugar ring at the 3' end of the first nucleic acid molecule is covalently connected to an amino group of the antibody via the first coupling agent. The sugar ring at the 5' end of the first nucleic acid molecule is not modified. Optionally, the sugar ring at the 3' end of the first nucleic acid molecule is modified with an NH2C7 modification group, and the NH2C7 modification group is covalently connected to the amino group of the antibody via the first coupling agent. Optionally, the first coupling agent is suberate bis(sulfosuccinimidyl) sodium salt.

In some embodiments of the present application, the 3' end of the second nucleic acid molecule is connected to a second fluorescent group. Optionally, the sugar ring at the 3' end of the second nucleic acid molecule is modified with a thiol group, the surface of the second fluorescent group is modified with an amino group, and the thiol group is covalently connected to the amino group on the surface of the second fluorescent group via a third coupling agent. Optionally, the third coupling agent is 4-(N-maleimidomethyl)cyclohexanecarboxylic acid N-hydroxysuccinimide ester.

In some embodiments of the present application, the sugar ring at the 5' end of the second nucleic acid molecule is covalently connected to the amino group of the scaffold protein of the substrate protein conjugate via the second coupling agent. Optionally, the sugar ring at the 5' end of the second nucleic acid molecule is modified with an NH2C6 modification group, and the NH2C6 modification group is covalently connected to the amino group of the scaffold protein via a second coupling agent. Optionally, the second coupling agent is suberate bis(sulfosuccinimidyl) sodium salt.

In some embodiments of the present application, the 5' end of the third nucleic acid molecule is covalently linked to the first fluorescent group. Optionally, the sugar ring at the 5' end of the third nucleic acid molecule is modified with an NH2C6 modification group, and the NH2C6 modification group is covalently connected to the first fluorescent group.

In some embodiments of the present application, the first hybridization zone is located upstream of the second hybridization zone according to the order from the 5' end to the 3' end in the first nucleic acid molecule. The third hybridization zone is located upstream of the fourth hybridization zone according to the order from the 5' end to the 3' end in the second nucleic acid molecule. In the third nucleic acid molecule, the fifth hybridization zone is located upstream of the sixth hybridization zone according to the order from the 5' end to the 3' end.

**In** some embodiments of the present application, the first nucleic acid molecule has 55 nucleotides, the first hybridization zone covers the 3rd to 10th base sites of the first nucleic acid molecule starting from the 5' end, and the second hybridization zone covers the 13th to 19th base sites from the 5' end of the first nucleic acid molecule. The second nucleic acid molecule has 53 nucleotides, the third hybridization zone covers 37th to 43rd base sites of the second nucleic acid molecule starting from the 5' end, and the fourth hybridization zone covers the 44th to 51st sites of the second nucleic acid molecule starting from the 5' end. The third nucleic acid molecule has 22 nucleotides. The fifth hybridization zone covers the 3rd to 10th base sites of the third nucleic acid molecule starting from the 5' end. The sixth hybridization zone covers the 11th to 18th base sites of the third nucleic acid molecule starting from the 5' end. The complementary pairing between the six DNA sequences causes the first nucleic acid molecule, the second nucleic acid molecule, and the third nucleic acid molecule to hybridize in pairs to form the stem-loop structure, and the shape of the stem is ⊥-shaped.

**In** some embodiments of the present application, the first hybridization zone is GCTGAGTT from the 5' end to the 3' end, and the sixth hybridization zone is AACTCAGC from the 5' end to the 3' end. The second hybridization zone is CAACGAC from the 5' end to the 3' end, and the third hybridization zone is GTCGTTG from the 5' end to the 3' end. The fourth hybridization zone is GCTGAGAT from the 5' end to the 3' end, and the fifth hybridization zone is ATCTCAGC from the 5' end to the 3' end. Each of the hybridization zone is the single-stranded DNA, rather than the double-stranded DNA. The two DNA sequences within the same single-stranded DNA do not pair with each other, but pair with the DNA sequences of the other single-stranded DNA.

**In** some embodiments of the present application, the full-length sequence of the first nucleic acid molecule is shown in SEQ ID No: 1, the full-length sequence of the second nucleic acid molecule is shown as SEQ ID No: 2, and the full-length sequence of the third nucleic acid molecule is shown as SEQ ID No: 3.

**In** some embodiments of the present application, G in the first nucleic acid molecule, the second nucleic acid molecule and/or the third nucleic acid molecule may be replaced by ᵢₛₒG, and C may be replaced by ᵢₛₒC. ᵢₛₒG and ᵢₛₒC are unnatural base pairs. Using the non-natural base pairs for pairing can effectively avoid mismatching between the first nucleic acid molecule, the second nucleic acid molecule and/or the third nucleic acid molecule and the natural nucleic acid in the solution to be tested, thereby preventing mismatching from affecting the formation of the stem-loop structure. This in turn avoids measurement error caused by mismatching.
the structural formula of ᵢₛₒG is: 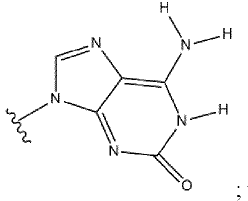 the broken line indicates a connection site.

The structural formula of ᵢₛₒC is:

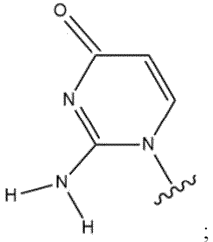

The bonding manner of ᵢₛₒG and ᵢₛₒC is: 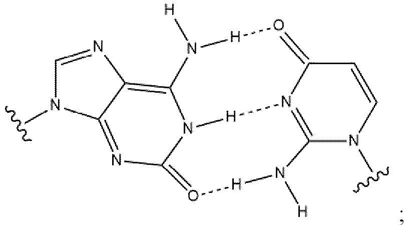 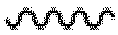 indicates connection to deoxyribose on the DNA molecule.

**In** some embodiments of the present application, G in the first hybridization zone and the sixth hybridization zone is replaced by ᵢₛₒG, and C is replaced by ᵢₛₒC. The first hybridization zone is ᵢₛₒGᵢₛₒCTᵢₛₒGAᵢₛₒGTT from the 5' end to the 3' end, and the sixth hybridization zone is AAᵢₛₒCTᵢₛₒCAᵢₛₒGᵢₛₒC from the 5' end to the 3' end.

**In** some embodiments of the present application, G in the second hybridization zone and the third hybridization zone is replaced by ᵢₛₒG, and C is replaced by ᵢₛₒC. The second hybridization zone is ᵢₛₒCAAᵢₛₒCᵢₛₒGAᵢₛₒC from the 5' end to the 3' end, and the third hybridization zone is ᵢₛₒGTᵢₛₒCᵢₛₒGTTᵢₛₒG from the 5' end to the 3' end.

**In** some embodiments of the present application, G in the fourth hybridization zone and the fifth hybridization zone is replaced by ᵢₛₒG, and C is replaced by ᵢₛₒC. The fourth hybridization zone is ᵢₛₒGᵢₛₒCTᵢₛₒGAᵢₛₒGAT from the 5' end to the 3' end, and the fifth hybridization zone is ATᵢₛₒCTᵢₛₒCAᵢₛₒGᵢₛₒC from the 5' end to the 3' end.

**In** some embodiments of the present application, the full-length sequence of the first nucleic acid molecule is:
AᵢₛₒCᵢₛₒGᵢₛₒCTᵢₛₒGAᵢₛₒGTTATᵢₛₒCAAᵢₛₒCᵢₛₒGAᵢₛₒCTTTTTTTATᵢₛₒCAᵢₛₒCATᵢₛₒCAᵢₛₒGᵢₛₒGᵢₛₒCTᵢₛₒCTAᵢₛₒG_{is o}CᵢₛₒGTATᵢₛₒGᵢₛₒCTATTᵢₛₒG. In other embodiments of the present application, the full-length sequence of the first nucleic acid molecule may be the sequence formed by replacing at least a part or all of G by ᵢₛₒG and replacing at least a part or all of C by ᵢₛₒC in the sequence shown in SEQ ID No: 1.

**In** some embodiments of the present application, the full-length sequence of the second nucleic acid molecule is:
TAᵢₛₒCᵢₛₒGTᵢₛₒCᵢₛₒCAᵢₛₒGAAᵢₛₒCTTTAᵢₛₒCᵢₛₒCᵢₛₒCᵢₛₒCᵢₛₒCAᵢₛₒCAᵢₛₒCᵢₛₒCᵢₛₒCTTTTTTTᵢₛₒGTᵢₛₒCᵢₛₒGTTᵢₛₒG_{i so}GᵢₛₒCTᵢₛₒGAᵢₛₒGATTᵢₛₒC. In other embodiments of the present application, the full-length sequence of the second nucleic acid molecule is the sequence formed by replacing at least a part or all of G by ᵢₛₒG and replacing at least a part or all of C by ᵢₛₒC in the sequence shown in SEQ ID No:2.

**In** some embodiments of the present application, the full-length sequence of the third nucleic acid molecule is:
ᵢₛₒCᵢₛₒGATᵢₛₒCTᵢₛₒCAᵢₛₒGᵢₛₒCAAᵢₛₒCTᵢₛₒCAᵢₛₒGᵢₛₒCAᵢₛₒGᵢₛₒCᵢₛₒG. In other embodiments of the present application, the full-length sequence of the third nucleic acid molecule is the sequence formed by replacing at least a part or all of G by ᵢₛₒG and replacing at least a part or all of C by ᵢₛₒC in the sequence shown in SEQ ID No:3.

**In** some embodiments of the present application, the oxidation inhibiting agent further includes a carrier molecule whose surface is bonded with the antioxidant. Because the first fluorescent group involves the oxidative luminescence instead of stimulated luminescence, the first fluorescent group may emit the fluorescence once there is the presence of the oxidizing substance. The function of the antioxidant is mainly to prevent the first fluorescent group from being oxidized by these substances with an oxidizing ability, thereby generating the background fluorescence that may affect the measurement result. These substances with oxidizing ability may come from the solution to be tested or a blood sample and the like. The antioxidant is selected from any one or more of cannabidiol, vitamin C, vitamin E, tea polyphenols, and glutathione. In some embodiments of the present application, the oxidant includes an alkaline solution of hydrogen peroxide. The oxidant may also be called as a chemiluminescent substrate of the first fluorescent group because these substrates themselves are oxidizing.

In some embodiments of the present application, the carrier molecule may be graphene oxide. A part of the carboxyl groups on graphene oxide is bound to the hydroxyl groups on the antioxidant via a sulfoxide oxide condensation agent, and a part of the carboxyl groups on graphene oxide is bound to the amino group on the antioxidant via 1- (3-dimethylaminopropyl) -3-ethylcarbodiimide hydrochloride, so that the antioxidant attaches to the graphene oxide. Because antioxidant and the oxidant cannot be added to the solution to be tested at the same time, otherwise the oxidant will not be able to oxidize the first fluorescent group to emit the light, the antioxidant needs to be removed from the solution to be tested before adding the oxidant. Binding the antioxidant to the graphene oxide carrier is more conducive to remove the antioxidant.

In various embodiments of the present application, two fluorescent groups are used to generate the second fluorescence for measurement, the first fluorescent group involves the oxidative luminescence, the second fluorescent group involves the luminescence by a light excitation, and the first fluorescence emitted by the first fluorescent group can excite the second fluorescent group to emit the second fluorescence on a basis of fluorescence resonance energy transfer. In the case that the second fluorescence is collected, it can be determined whether there is a small molecule substance in the solution to be tested or it is possible to obtain the concentration of the small molecule substance. If the intensity of the second fluorescence reaches the maximum value, it means that the free small molecule substance will not competitively bind the antibody with the small molecule substrate in the bound state, so the solution to be tested does not contain the small molecule substance at all. In addition, since the content of the small molecule substance is inversely proportional to the intensity of the second fluorescence, the content of the small molecule substance can also be obtained on a basis of the intensity of the second fluorescence. This detection method eliminates the influence of the background fluorescence of the first fluorescent group on the measurement result and improves the sensitivity and accuracy of the detection. In addition, the two fluorescent groups in various embodiments of the present application can emit the fluorescence signal without needing to set up the additional excitation light source, which also reduces the complexity of the detection system and enables to achieve mobile detection. Furthermore, the first fluorescent group in each embodiment of the present application involves the flashing luminescence, has a short detection time and can quickly obtain the detection result.

Some embodiments of the present application also provide a method for detecting a small molecule substance, which includes the following steps:
(1) adding the first conjugate, the second conjugate, the third conjugate and the oxidation inhibiting reagent are added to the solution to be tested, and performing mixing to form the sample to be tested.
   In some embodiments, in step (1), the first conjugate is formed at least by coupling a first nucleic acid molecule to an antibody. The first nucleic acid molecule contains a first hybridization zone and a second hybridization zone. The antibody can specifically bind to the small molecule substance. The second conjugate is formed at least by sequentially coupling a substrate protein conjugate, a second nucleic acid molecule and a second fluorescent group. The second nucleic acid molecule has a third hybridization zone and a fourth hybridization zone. The third hybridization zone is complementary to the second hybridization zone. The small molecule substrate of the substrate protein conjugate can specifically bind to the complementarity determining region of the antibody. The third conjugate is formed at least by coupling a first fluorescent group to a third nucleic acid molecule. The third nucleic acid molecule contains a fifth hybridization zone and a sixth hybridization zone, the fifth hybridization zone is complementary to the fourth hybridization zone, and the sixth hybridization zone is complementary to the first hybridization zone. In the condition that the solution to be tested does not contain the free small molecule substance or contains the small molecule substance at a low concentration, the antibody forms an immune complex with the substrate protein conjugate by binding to the small molecule substance, and the first nucleic acid molecule , the second nucleic acid molecule and the third nucleic acid molecule form the stem-loop structure, and the first fluorescent group and the second fluorescent group are located on the same side of the stem-loop structure, so the fluorescence resonance energy transfer can occur between the two fluorescent molecules, thus, the second fluorescent group is excited to emit the second fluorescence.
(2) removing the oxidation inhibiting reagent used to inhibit the first fluorescent group from being oxidized to emit the fluorescence from the sample to be tested, adding an oxidant used to oxidize the first fluorescent group to emit the first fluorescence, and collecting the second fluorescence at the maximum emission wavelength of the second fluorescent group, that is, excluding the fluorescence at other wavelengths than the maximum emission wavelength.
(3) In a case that the second fluorescence is collected, obtaining the content of the small molecule substance on a basis of the intensity of the second fluorescence and the one-to-one correspondence between the intensity of the fluorescence and the content of the small molecule substance.

When collecting the intensity of the second fluorescence, in order to avoid the interference of the first fluorescence on the second fluorescence, an optical filter may be used to filter out the fluorescence generated after the first fluorescent group is oxidized, and the second fluorescence is only allowed to pass through the optical filter, thereby collecting the second fluorescence emitted by the second fluorescent group, and obtaining the content of the small molecule substance on a basis of the intensity of the second fluorescence.

The solution to be tested may be derived from a blood sample. The composition of the blood is relatively complex and contains a variety of oxidative substances. If the first fluorescent group is oxidized by these oxidative substances, the background fluorescence will be generated, which will affect the accuracy of the measurement result. Therefore, in the present application, in order to reduce the influence of the background fluorescence on the measurement result, the first fluorescence emitted by the first fluorescent group is not used as the detection fluorescence signal, and instead the second fluorescence emitted by the second fluorescent group is used as the detection fluorescence signal.

In some embodiments of the present application, the working concentration of the first conjugate may range from 1 nM to 20 nM in the sample to be tested. The working concentration of the second conjugate may range from 1 nM to 20 nM. The working concentration of the third conjugate may range from 0.05 nM to 0.2 nM. The working concentration of the oxidation inhibiting reagent may range from from 15 µg/ml to 25 µg/ml.

In some embodiments of the present application, the solution to be tested is derived from the blood sample such as a whole blood sample, a serum sample, or a plasma sample.

In some embodiments of the present application, the mixing duration time may be from 5 minutes to 10 minutes.

In some embodiments of the present application, the mixing temperature may be 36 to 37 degrees.

In some embodiments of the present application, the volume of the oxidant may be 200 µL, the oxidant is an alkaline hydrogen peroxide solution, and the pH value is 8.0. The alkaline hydrogen peroxide solution is prepared by dissolving hydrogen peroxide in TBS buffer, where the final concentration of hydrogen peroxide is 0.1M and the final concentration of TBS is 10mM.

In some embodiments of the present application, the small molecule substance includes triiodothyronine, tetraiodothyronine, or progesterone.

In some embodiments of the present application, in the mixed sample to be tested, in the condition that the solution to be tested does not contain or contains a small amount of the small molecule substance, the antibody in the first conjugate forms an immune complex with the small molecule substrate. The immune complex brings the distance of the complementary sequences of the three single-stranded DNAs closer to complementarily pair them pairwise and achieve assembly between the DNAs, thereby forming a stem-loop structure.

Some embodiments of the present application also provide a kit for detecting a small molecule substance, which includes: a first storage tube, a second storage tube, a third storage tube, a fourth storage tube and a fifth storage tube.

where, the first storage tube stores at least the conjugate of the first nucleic acid molecule and the antibody.

The second storage tube stores at least the conjugate of the substrate protein conjugate, the second nucleic acid molecule and the second fluorescent group. The antibody and the substrate protein conjugate can form an immune complex with the small molecule substrate in the absence of the small molecule substance or presence of a small amount of the small molecule substance. On the contrary, if there is an excess of the small molecule substance, then the small molecule substance will competitively bind to the antibodies that originally bind to the small molecule substrate, and the immune complex will not be formed, thus not causing the assembly between the single-stranded DNAs.

The third storage tube stores at least the conjugate of the first fluorescent group and the third nucleic acid molecule. The first nucleic acid molecule, the second nucleic acid molecule and the third nucleic acid molecule are assembled into a stem-loop structure on a basis of the proximity effect under the condition that the immune complex is formed. The spatial conformation of the stem-loop structure makes the first fluorescent group and the second fluorescent group spontaneously arranged on the same side of the stem-loop structure, and the spatial distance between the two is smaller than the limit spacing distance at which the fluorescence resonance energy transfer can occur, for example, in the range of 70 angstroms to 99 angstroms, which provides the conditions for occurrence of the fluorescence resonance energy transfer between the two fluorescent groups.

The fourth storage tube stores at least an antioxidant capable of inhibiting the first fluorescent group from being oxidized. Optionally, the antioxidant may be modified on the carrier molecule to become an oxidation inhibiting reagent. Optionally, the carrier molecule may be selected from graphene oxide.

The fifth storage tube stores at least the oxidant. The oxidant is capable of oxidizing the first fluorescent group to emit the first fluorescence. The oxidant and the antioxidant are not present in the sample to be tested at the same time. Before adding the oxidant, the antioxidant and the carrier molecule need to be removed from the sample to be tested. Since the antioxidant is modified on the carrier molecule, and the carrier molecule graphene oxide exhibits a lamellar network structure and is easily removed from the sample to be tested, the removal of the antioxidant can be achieved by removing the carrier molecule. In addition, the graphene oxide can also adsorb the free third nucleic acid molecule, thereby adsorbing the conjugate of the first fluorescent group and the third nucleic acid molecule, so that the antioxidant inhibits the first fluorescent group in the free state from being improperly oxidized, thus suppressing the first fluorescent group from the inappropriate emission of the background fluorescence.

In the embodiments of the present application, the first nucleic acid molecule contains a first hybridization zone and a second hybridization zone; The second nucleic acid molecule has a third hybridization zone and a fourth hybridization zone; The third nucleic acid molecule contains a fifth hybridization zone and a sixth hybridization zone; The third hybridization zone is complementary to the second hybridization zone, the fifth hybridization zone is complementary to the fourth hybridization zone, and the sixth hybridization zone is complementary to the first hybridization zone. At least a part or all of the first hybridization zone, the second hybridization zone, the third hybridization zone, the fourth hybridization zone, the fifth hybridization zone and the sixth hybridization zone contain the bases ᵢₛₒG and ᵢₛₒC. Since ᵢₛₒG is used to replace the natural base G and ᵢₛₒC is used to replace the natural base C, the possibility of mismatching is reduced.

In the embodiment of the present application, the detection principle of the kit for detecting the small molecule substance is as follows.

In the condition that the solution to be tested does not contain the small molecule substance or contains a small amount of the small molecule substance, the antibody and the substrate protein conjugate form an immune complex in whole or in part. The first nucleic acid molecule, the second nucleic acid molecule and the third nucleic acid molecule are close to each other, hybridized and self-assembled into the stem-loop structure. Six DNA sequences (i.e., the hybridization zone) in three nucleic acid molecules (i.e., the single-stranded DNA) are complementarily paired partwise to form the neck portion of the stem-loop structure, and the remaining non-DNA sequences form the ring portion of the stem-loop structure. The two DNA sequences inside each of the single-stranded DNAs cannot pair complementarily, but can only pair with the complementary sequence of another single-stranded DNA. The spatial conformation of the stem-loop structure causes the first fluorescent group and the second fluorescent group to be located on the same side of the stem-loop structure, and the distance between them can ensure that the fluorescence resonance energy transfer occurs between them. In the condition that the oxidation inhibiting reagent is removed, the oxidant can oxidize the first fluorescent group and cause it to emit the first fluorescence. The first fluorescence excites the second fluorescent group to emit the second fluorescence on a basis of the fluorescence resonance energy transfer effect to obtain the content of the small molecule substance according to the intensity of the second fluorescence.

In the condition that the solution to be tested contains an excess amount of the small molecule substance, the immune complex and the stem-loop structure cannot be formed. Even if the antioxidant is removed and the subsequent oxidant is added to cause the first fluorescent group to emit the first fluorescence, the first fluorescent group cannot excite the second fluorescent group to emit the second fluorescence since the distance between the fluorescent group and the second fluorescent group is greater than the limit spacing distance at which fluorescence resonance energy transfer can occur. The inability to obtain the intensity of the second fluorescence indicates that the solution to be tested contains an excess amount of the small molecule substance.

In the embodiments of the present application, the second fluorescent group can only emit the second fluorescence under the excitation of the excitation light. Without the excitation of the first fluorescence, the second fluorescent group will not emit the second fluorescence for measuring the content of the small molecule substance. Therefore, the second fluorescence group as the detection group will basically not emit the background fluorescence, which ensures the reliability of using the second fluorescence as the detection fluorescence.

Some embodiments of the present application provide a system for detecting a small molecule substance, which includes: a reaction vessel, a micro-syringe pump, an optical filter, an optical signal detection module and a calculation module.

The reaction vessel has an accommodation chamber capable of accommodating the solution to be tested.

The microinjection pump is communicated with to the accommodation chamber through an injection pipeline, and injects the mixture of the first conjugate, the second conjugate, the third conjugate and the oxidation inhibiting reagent into the accommodation chamber through the injection pipeline to mix with the solution to be tested; The first conjugate is formed at least by coupling a first nucleic acid molecule to an antibody, the second conjugate is formed at least by sequentially coupling a substrate protein conjugate, a second nucleic acid molecule and a second fluorescent group, and the third conjugate is formed at least by coupling a first fluorescent group to a third nucleic acid molecule, and the oxidation inhibiting reagent contains an antioxidant that inhibits the first fluorescent group from being oxidized to emit the first fluorescence.

The optical filter is disposed on an exit light path of the first fluorescence and allows the transmission of the second fluorescence having the same wavelength as the maximum emission wavelength of the second fluorescence group.

The optical signal detection module is disposed on the exit light path of the first fluorescence and is located on the downstream side of the optical filter, and acquires the second fluorescence transmitted via the optical filter. The intensity value of the second fluorescence may be 0, which indicates that there is an excess amount of the small molecule substance in the solution to be tested, because the second fluorescent group involves luminescence by excitation rather than oxidative luminescence; in the condition that the second fluorescent group is not excited by the excitation light, it does not emit the second fluorescence by itself, so it will not produce the background fluorescence noise. Therefore, in the presence of the excess amount of the small molecule substance, the intensity of the second fluorescence may reach zero, and in the absence of the small molecule substance or in the presence of a small amount of the small molecule substance, there will be the second fluorescence. The intensity value of the second fluorescence is inversely proportional to the content of the small molecule substance. If the first fluorescence is used as the detection fluorescence, since the first fluorescence is oxidative luminescence, and there will be the oxidative substances in the solution to be tested, the first fluorescence will be oxidized by these oxidative substances and emit the background fluorescence noise. Therefore, using the second fluorescence as the detection fluorescence is more accurate and has smaller error than using the first fluorescence as the detection fluorescence.

The calculation module converts the second fluorescence into a digital signal and obtains the content of the small molecule substance in the solution to be tested according to the functional relationship between the intensity of the fluorescence and the content of the small molecule substance.

Due to the adoption of the above technical solutions, some embodiments of the present application can achieve the following technical effects:

Some embodiments of the present application use a combination of immune reaction and fluorescence resonance energy transfer effect to detect whether the solution to be tested contains the small molecule substance. Specifically:

In the condition that the solution to be tested does not contain the small molecule substance or contains a small amount of the small molecule substance, the antibody and the substrate protein conjugate form an immune complex on a basis of the immune reaction, so that the distance between the first nucleic acid molecule, the second nucleic acid molecule and the third nucleic acid molecule is drawn closer, causing DNA assembly and forming the stem-loop structure. The stem-loop structure causes the first fluorescent group and the second fluorescent group to be located on the same side of the stem-loop structure, thereby generating a fluorescence resonance energy transfer phenomenon between the two and causing the second fluorescent group to emit the second fluorescence as a fluorescence signal to be measured. The content of the small molecule substance in the solution to be tested is obtained according to the preset functional relationship between the intensity of the fluorescence and the content of the small molecule substance and the intensity of the fluorescence signal to be measured.

In the condition that there is an excess amount of the small molecule substance in the solution to be tested, the immune complex and the stem-loop structure cannot be formed, and the second fluorescent group will not be excited by the excitation light (i.e. the first fluorescence) to emit the second fluorescence, then the fluorescence signal to be measured can't be captured, indicating that there is an excess amount of the small molecule substance in the solution to be tested. In addition, even if the first fluorescent group emits the background fluorescence under the action of certain oxidizing substances, since the stem-loop structure cannot be formed, the spatial distance between the first fluorescent group and the second fluorescent group is larger than the minimum distance required by the fluorescence resonance energy transfer effect and the fluorescence resonance energy transfer effect will not occur between the two fluorescent groups, and the second fluorescent group will not emit the second fluorescence on a basis of the background fluorescence. As such, this at least enables to achieve qualitative detection of the presence or absence of the small molecule substance in the solution to be tested and quantitative detection of the content of the small molecule substance in the solution to be tested.

Various embodiments of the present application use the second fluorescence emitted by the second fluorescent group that is less likely to generate the background fluorescence noise as the fluorescence signal to be measured, instead of using the first fluorescence emitted by the first fluorescent group that usually has the background fluorescence noise as the fluorescence signal to be measured, which can greatly reduce the impact of the background fluorescence noise on detection results, improve the detection sensitivity and reduce the detection error, making the measured protein content closer to the true value.

### DESCRIPTION OF THE DRAWINGS

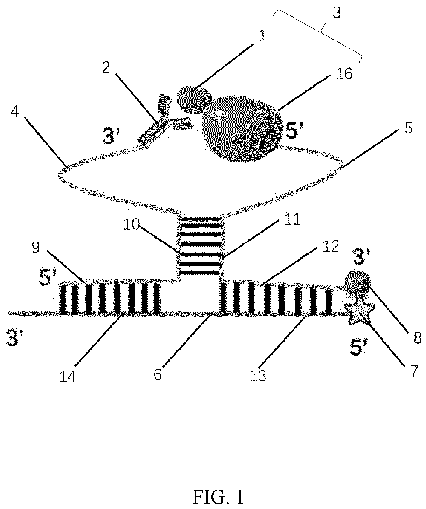

FIG. 1 is a schematic diagram of the immune complex and the stem-loop structure of some embodiments of the present application.

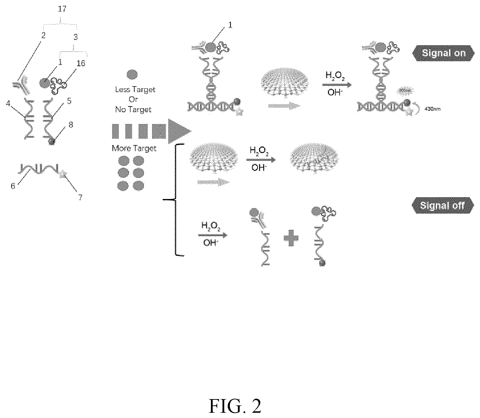

FIG. 2 is a schematic diagram of a detection method according to some embodiments of the present application. In FIG. 2, when there is no small molecule substance or a small amount of the small molecule substance (Target shown in FIG. 2) in the solution to be tested, the antibody and the small molecule substrate will form an immune complex, and a stem-loop structure is formed between the single-stranded DNA molecules. After eluting off the graphene oxide modified with the antioxidant, an oxidant (an alkaline solution of hydrogen peroxide) is added. The acridinium ester is oxidized by the oxidant to emit the first fluorescence of 430nm. The first fluorescence excites the quantum dot to emit the second fluorescence of 605nm. At this time, this is referred to as Signal on state. When there is an excess amount of the small molecule substance, no immune complex and stem-loop structure are produced. The third nucleic acid molecule is adsorbed on the surface of graphene oxide via π-π stacking effect, and when the graphene oxide modified with the antioxidant is eluted off, the conjugate of the third nucleic acid molecule will be eluted together along with the graphene oxide and remain in the eluted liquid. The acridinium ester will be oxidized by the oxidizing substance in the liquid to produce the background fluorescence, and the remaining liquid after elution contains the substrate protein conjugate and the antibodies bound to the small molecule substance. The quantum dot does not produce fluorescence at least because they are not excited by excitation light. At this time, this is called as the Signal off state, indicating that there is an excess amount of the small molecule substance in the liquid.

### Reference symbols:

Small molecule substance 1, Antibody 2, Substrate protein conjugate 3, First nucleic acid molecule 4, Second nucleic acid molecule 5, Third nucleic acid molecule 6, First fluorescent group 7, Second fluorescent group 8, First hybridization zone 9, Second hybridization zone 10, Third hybridization zone 11, Fourth hybridization zone 12, Fifth hybridization zone 13, Sixth hybridization zone 14, Composite of graphene oxide and antioxidant 15 (i.e., Oxidation inhibiting reagent), Scaffolding protein 16, Immune complex 17.

### DETAILED DESCRIPTION

The technology of each embodiment of the present application is described in detail below with reference to specific embodiments. It should be understood that the following specific embodiments are only used to help those skilled in the art understand the present application, but are not intended to limit the present application. In addition, the following specific embodiments can be combined arbitrarily to form new examples without exerting any creative effort.

### [Reagent combination for detecting the small molecule substance]

Some embodiments of the present application provide a reagent combination. The reagent combination is used to detect whether there is excess amount of the small molecule substance in the solution to be tested, thereby achieving qualitative detection of the small molecule substance. Moreover, the reagent combination can also obtain the concentration of the small molecule substance in the solution to be tested on the premise that there is a small amount of the small molecule substance in the solution to be tested, thereby achieving quantitative detection of the content of the small molecule substance.

In some embodiments of the present application, the solution to be tested may be derived from a blood sample or other non-blood sample. the blood sample may be derived from a whole blood sample, a serum sample or a plasma sample.

In some embodiments of the present application, the types of the small molecule substance to be detected are not particularly limited. Exemplarily, the small molecule substance may be triiodothyronine, tetraiodothyronine, or progesterone, etc. In some embodiments of the present application, the molecular weight of the small molecule substance to be detected may be in the range of 100 to 5000 KD, but in some cases it is not limited to the above range.

In some embodiments of the present application, the reagent combination includes: a first conjugate, a second conjugate, a third conjugate and an oxidation inhibiting reagent.

the first conjugate is formed at least by coupling a first nucleic acid molecule to an antibody. The sugar ring at the 3' end of the first nucleic acid molecule is modified with an NH2C7 modification group, and the NH2C7 modification group is covalently connected to the amino group of the antibody via the first coupling agent. The NH2C7 modification group modifies the sugar ring at the 3' end of the first nucleic acid molecule, rather than the base. The first coupling agent may be suberate bis(sulfosuccinimidyl) sodium salt. The amino group connected to the first coupling agent in the antibody is not located on the CDR of the antibody, otherwise the antibody cannot specifically bind to the small molecule substance. The 3' end of the first nucleic acid molecule is not modified.

In some embodiments, the full-length sequence of the first nucleic acid molecule is shown as SEQ ID No: 1. The specific sequence may be ACGCTGAGTTATCAACGACTTTTTTTATCACATCAGGCTCTAGCGTATGCTATTG, but is not limited to the above sequence.

In some embodiments, the first nucleic acid molecule contains a first hybridization zone and a second hybridization zone. In the first nucleic acid molecule, the first hybridization zone may be located upstream of the second hybridization zone according to the order from the 5' end to the 3' end. Exemplarily, the first hybridization zone may be GCTGAGT from the 5' end to the 3' end, and the second hybridization zone may be CAACGAC from the 5' end to the 3' end. However, the complementary pairing does not occur between the first hybridization zone and the second hybridization zone. In various embodiments of the present application, the complementary pairing does not occur between the two DNA sequences contained in each single-stranded DNA. Only the single-stranded DNA between different reagents undergo the complementary pairing to assemble into the stem-loop structure.

In some embodiments, the first nucleic acid molecule has 55 nucleotides, the first hybridization zone covers the 3rd to 10th base sites of the first nucleic acid molecule starting from the 5' end, and the second hybridization zone covers the 13rd to 19th base sites of the first nucleic acid molecule starting from the 5' end.

In some embodiments, the antibody can specifically bind to the small molecule substance. Each antibody molecule can only bind to one small molecule substance, but cannot bind to multiple small molecule substance at the same time. Moreover, the CDR of the antibody molecule can only recognize one antigenic epitope of the small molecule substance, so the specific binding of the two can be achieved.

The second conjugate may be formed at least by sequentially coupling a substrate protein conjugate, a second nucleic acid molecule and a second fluorescent group.

In some embodiments, the substrate protein conjugate is formed at least by coupling the small molecule substrate to the scaffold protein. The small molecule substrate is the same as the small molecule substance, so the small molecule substrate and the small molecule substance can competitively bind to the same antibody. When the concentration of the small molecule substance is very high, the small molecule substrate can be rendered basically not bind to the CDR of the antibody. Therefore, when there is no the small molecule substance or a small amount of the small molecule substance in the solution to be tested, the antibody and the substrate protein conjugate can undergo an immune reaction to form an immune complex.

In some embodiments, the full-length sequence of the second nucleic acid molecule is shown as SEQ ID No: 2. The specific sequence may be TACGTCCAGAACTTTACCAAACCACACCCTTTTTTTGTCGTTGGCTGAGATTC, but is not limited to the above sequence.

In some embodiments, the second nucleic acid molecule has a third hybridization zone and a fourth hybridization zone. In the second nucleic acid molecule, the third hybridization zone is located upstream of the fourth hybridization zone according to the order from the 5' end to the 3' end. Exemplarily, the third hybridization zone is GTCGTTG from the 5' end to the 3' end, and the fourth hybridization zone is GTCTGAGAT from the 5' end to the 3' end. The third hybridization zone is complementary to the second hybridization zone. There is no complementary relationship between the third hybridization zone and the fourth hybridization zone, therefore, the possibility of mismatching within the DNA strands is reduced.

In some embodiments, the second nucleic acid molecule has 53 nucleotides. The third hybridization zone covers the 37th to 43rd base sites of the second nucleic acid molecule started from the 5' end, and the fourth hybridization zone covers the 44th to 51st base sites of the second nucleic acid molecule started from the 5' end.

In some embodiments, the 3' end of the second nucleic acid molecule is connected to a second fluorescent group. Exemplarily, the sugar ring at the 3' end of the second nucleic acid molecule is modified with a thiol group, the surface of the second fluorescent group is modified with an amino group, and the thiol group is covalently connected to the amino group on the surface of the second fluorescent group via a third coupling agent. The third coupling agent may be 4-(N-maleimidomethyl)cyclohexanecarboxylic acid N-hydroxysuccinimide ester.

In some embodiments, the sugar ring at the 5' end of the second nucleic acid molecule is modified with an NH2C6 modification group, and the NH2C6 modification group is covalently connected to the amino group of the substrate protein conjugate via a second coupling agent. The second coupling agent is suberate bis(sulfosuccinimidyl) sodium salt. The amino group for connecting the scaffold protein to the second coupling agent in the substrate protein conjugate is not located at the binding site between the scaffold protein and the small molecule substrate, otherwise, the scaffold protein cannot specifically bind to the small molecule substrate.

The third conjugate may be formed at least by coupling the first fluorescent group to the third nucleic acid molecule.

In some embodiments, the sugar ring at the 5' end of the third nucleic acid molecule is modified with an NH2C6 modification group, and the NH2C6 modification group is covalently connected to the first fluorescent group.

In some embodiments, the full-length sequence of the third nucleic acid molecule is shown as SEQ ID No: 3. The specific sequence may be CGATCTCAGCAACTCAGCAGCG, but is not limited to the above sequence.

In some embodiments, the third nucleic acid molecule contains a fifth hybridization zone and a sixth hybridization zone, and the fifth hybridization zone and the sixth hybridization zone are not complementary to each other.

In some embodiments, in the third nucleic acid molecule, the fifth hybridization zone is located upstream of the sixth hybridization zone according to the order from the 5' end to the 3' end.

In some embodiments, the third nucleic acid molecule has 22 nucleotides, the fifth hybridization zone covers the 3rd to 10th base sites of the third nucleic acid molecule starting from the 5' end, and the sixth hybridization zone covers the 11th to 18th base sites of the third nucleic acid molecule starting from the 5' end. The sequence of the fifth hybridization zone from the 5' end to the 3' end may be ATCTCAGC. The sequence of the sixth hybridization zone from the 5' end to the 3' end may be AACTCAGC.

In some embodiments, the fifth hybridization zone is complementary to the fourth hybridization zone, the sixth hybridization zone is complementary to the first hybridization zone, and the third hybridization zone is complementary to the second hybridization zone. When the solution to be tested does not contain or contains a small amount of the small molecule substance, the antibody binds to the substrate protein conjugate to form an immune complex. The formation of the immune complex will drive conformational changes and DNA assembly between the three single-stranded DNAs, and with the help of the complementary pairing relationship between the above-mentioned DNA sequences, the stem-loop structure can be induced formation between the three single-stranded DNAs. The first fluorescent group and the second fluorescent group are located on the same side of the stem-loop structure, and the fluorescence resonance energy transfer (FRET) occurs between the two fluorescent molecules.

In some embodiments, the first fluorescent group emits the first fluorescence in the condition that it can be oxidized by an oxidant, and the first fluorescence excites the second fluorescent group to emit the second fluorescence on a basis of fluorescence resonance energy transfer effect in the condition that the first nucleic acid molecule, the second nucleic acid molecule, and the third nucleic acid molecule are paired with each other, so as to obtain the content of the small molecule substance on a basis of the intensity of the second fluorescence. When there is no or a small amount of small molecule substance in the solution to be tested, since the distance between the first fluorescent group and the second fluorescent group satisfies the conditions for the generation of fluorescence resonance energy transfer, the second fluorescent group will produce the second fluorescence, the wavelength of the second fluorescence is different from that of the first fluorescence. Therefore, using the second fluorescence as the detection fluorescence will not be affected by the background fluorescence emitted by the first fluorescent group. If the intensity of the second fluorescence is not obtained from the solution to be tested, it means that there is an excess of the small molecule substance in the solution to be tested. When there is no or a small amount of the small molecule substance in the solution to be tested, the antibody binds to the small molecule substance to form a stable immune complex, and three single-stranded DNAs form a stem-loop structure. The immune complex and the stem-loop structure can make the distance between the first fluorescent group and the second fluorescent group sufficient to produce the fluorescence resonance energy transfer effect, thereby determining the content of the small molecule substance according to the standard curve between the intensity and intensity content of the second fluorescence emitted by the second fluorescent group.

In some embodiments, the first fluorescent group emits the fluorescence without requiring irradiation of the excitation light, but emits the light under the oxidation effect of the oxidant. However, the second fluorescent group needs to be irradiated by the excitation light to emit the fluorescence. Without the irradiation of the excitation light, the second fluorescent group itself will not actively emit the fluorescence, so it is not easy to generate the background fluorescence. Therefore, the measurement result of the embodiments in the present application are not interfered by the background fluorescence of the second fluorescent group. Compared with the method of using only one fluorescent group to emit the fluorescence, the method of various embodiments of the present application can effectively avoid the interference of the background fluorescence of the fluorescent molecule, so the measurement result is more accurate.

In some embodiments, during detection, the first fluorescent group and the second fluorescent group need to be able to undergo the fluorescence resonance energy transfer, and the following conditions need to be met: The first conjugate, the second conjugate, and the third conjugate are completely complementarily paired in the absence of the small molecule substance, and partially complementarily paired in the presence of a small amount of the small molecule substance. Complementary pairing can form the stem-loop structure such that the spacing distance between the first fluorescent group and the second fluorescent group is less than or equal to 100 Angstroms. At this time, the first fluorescence emitted by the first fluorescent group can become the excitation light of the second fluorescent group, thereby exciting the second fluorescent group to generate the second fluorescence (as shown in FIGS. 1 and 2).

In some embodiments, the first fluorescent group may be an acridinium ester. Acridinium ester emits the fluorescence without requiring the excitation light, but instead achieves oxidative luminescence in the presence of the oxidant. Since there may be the oxidizing substance in the solution to be tested, it can also oxidize acridinium ester and make it emit the light. Therefore, acridinium ester will result in the background fluorescence. If the fluorescence of acridinium ester is used as the measured fluorescence, the background fluorescence will interfere with the measured fluorescence. Therefore, in order to further improve the sensitivity of detection and reduce the measurement error, the embodiments of the present application do not use the fluorescence of acridinium ester as the measured fluorescence. The chemiluminescence substrate of acridinium ester is an alkaline solution of H₂O₂, also known as an oxidant. When acridinium ester and the alkaline solution of H₂O₂ coexist, the molecules of acridinium ester are attacked by hydrogen peroxide ions. Acridinium ester can form unstable dioxyethane with hydrogen peroxide (H₂O₂), which then breaks down to emit the fluorescence.

In some embodiments, the maximum emission wavelength of the first fluorescent group acridinium ester is 430 nm. In some embodiments, the second fluorescent group may be a quantum dot. The maximum absorption wavelength of the second fluorescent group quantum dot may be in the range of 420 nm to 520 nm, for example, it may be 470 nm. The maximum emission wavelength of the second fluorescent group may be in the range of 595 nm to 615 nm, for example, it may be 605 nm. Therefore, the first fluorescent group and the second fluorescent group in the embodiments of the present application can produce the fluorescence resonance energy transfer effect. In some embodiments, the quantum dot is the core-shell structure quantum dot, the core layer material of which is selected from one or more of CdSe, CdS, CdTe, CdSeTe, CdZnS, ZnTe, CdSeS, PbS and PbTe, and the shell layer material of which is selected from one or more of ZnS, ZnSe, ZnSeS, PbS and PbSeS. In some embodiments, the particle size of the quantum dot may range from 3 to 5 nm, exemplarily, it may be 4.1 nm.

In some embodiments, G in the first nucleic acid molecule, the second nucleic acid molecule and the third nucleic acid molecule may be replaced by ᵢₛₒG, and C may be replaced by ᵢₛₒC. Since the natural nucleic acids also exist in these samples when the solution to be tested comes from the blood sample etc., the first nucleic acid molecule, the second nucleic acid molecule and the third nucleic acid molecule can be prevented from specifically binding to the nucleic acid in the blood sample by introducing the non-natural base pairs (ᵢₛₒG and ᵢₛₒC), thereby avoiding the measurement error caused due to DNA mismatch. In addition, these unnatural base pairs do not affect the mutual pairing of the first nucleic acid molecule, the second nucleic acid molecule, and the third nucleic acid molecule, and can still form a stable stem-loop structure, thereby not affecting the generation of the second fluorescence. Exemplarily, in the full-length sequence of the first nucleic acid molecule, G is replaced by ᵢₛₒG and C is replaced by ᵢₛₒC. In the full-length sequence of the second nucleic acid molecule, G is replaced by ᵢₛₒG and C is replaced by ᵢₛₒC. In the full-length sequence of the third nucleic acid molecule, G is replaced by ᵢₛₒG and C is replaced by ᵢₛₒC.

In some embodiments, G in the first hybridization zone and the sixth hybridization zone paired with each other are replaced by ᵢₛₒG, and C is replaced by ᵢₛₒC. Exemplarily, the first hybridization zone is ᵢₛₒGᵢₛₒCTᵢₛₒGAᵢₛₒGTT from the 5' end to the 3' end, and the sixth hybridization zone is AAᵢₛₒCTᵢₛₒCAᵢₛₒGᵢₛₒC from the 5' end to the 3' end.

In some embodiments, G in the second hybridization zone and the third hybridization zone paired with each other are replaced by ᵢₛₒG, and C is replaced by ᵢₛₒC. Exemplarily, the second hybridization zone is ᵢₛₒCAAᵢₛₒCᵢₛₒGAᵢₛₒC from the 5' end to the 3' end, and the third hybridization zone is ᵢₛₒGTᵢₛₒCᵢₛₒGTTᵢₛₒG from the 5' end to the 3' end.

In some embodiments, G in the fourth and fifth hybridization zones paired with each other is replaced by ᵢₛₒG, and C is replaced by ᵢₛₒC. Exemplarily, the fourth hybridization zone is ᵢₛₒGᵢₛₒCTᵢₛₒGAᵢₛₒGAT from the 5' end to the 3' end, and the fifth hybridization zone is ATᵢₛₒCTᵢₛₒCAᵢₛₒGᵢₛₒC from the 5' end to the 3' end.

In the above embodiment, the structural formula of ᵢₛₒG may be:

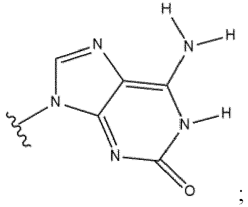

the structural formula of ᵢₛₒC may be:

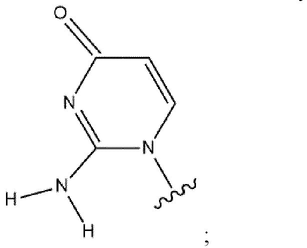

The bonding manner of ᵢₛₒG and ᵢₛₒC may be: 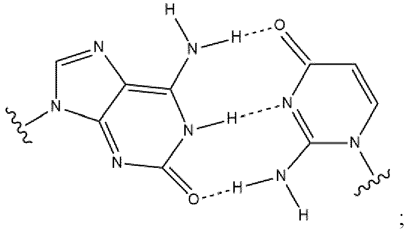 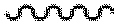 indicates linkage with deoxyribose on DNA molecule.

Therefore, the complementary pairing can be produced between ᵢₛₒG and ᵢₛₒC, which does not affect the mutual pairing of the first nucleic acid molecule, the second nucleic acid molecule and the third nucleic acid molecule, but can prevent the mismatch of the first nucleic acid molecule, the second nucleic acid molecule and the third nucleic acid molecule with the natural nucleic acid in the solution to be tested. Therefore, the above embodiment can reduce the measurement error caused due to the mismatch between the single-stranded DNA and the natural nucleic acid molecule.

The oxidation inhibiting reagent includes an antioxidant used for preventing the first fluorescent group from being oxidized to emit the first fluorescence. During detection, the first conjugate, the second conjugate, the third conjugate and the oxidation inhibiting reagent need to be added to the solution to be tested, and then mixed to form the sample to be tested. When there is the antioxidant in the sample to be tested, even if the first conjugate, the second conjugate, and the third conjugate form the stem-loop structure, the first fluorescent group acridinium ester will not emit the fluorescence because the antioxidant inhibits the oxidative luminescence of acridinium ester. Therefore, the antioxidant needs to be removed from the sample to be tested before testing.

In some embodiments, in order to achieve smooth removal of the antioxidant from the sample to be tested, the oxidation inhibiting reagent further includes a carrier molecule and binds the antioxidant to the surface of the carrier molecule. At this time, since the carrier molecule has a larger molecular weight, it is more easily removed from the sample to be tested. If only the carrier molecule is removed, it is possible to achieve simultaneous removal of the antioxidant.

In some embodiments, the carrier molecule may be graphene oxide (GO). The carboxyl group on graphene oxide is bound to the hydroxyl group on the antioxidant via a sulfoxide oxide condensation agent, and the carboxyl group on graphene oxide is bonded to the amino group on the antioxidant via 1-(3-dimethylaminopropyl)-3-ethylcarbodiimide hydrochloride. The first conjugate, the second conjugate, the third conjugate and the oxidation inhibiting reagent are added to the solution to be tested to form the sample to be tested. The third nucleic acid molecule is adsorbed on the surface of graphene oxide via π-π stacking function. The acridinium ester (AE) terminally labelled thereof cannot be oxidized to emit light due to the presence of the antioxidant. Even if a small part of the acridinium ester results in chemiluminescence, that is, produces the background fluorescence at a wavelength of 430nm, it will not affect the measurement result. This is because the fluorescence obtained during detection comes from the second fluorescent group quantum dot without coming from acridinium ester, the optical filter may be disposed to only obtain the fluorescence emitted by the quantum dot and filter out the background fluorescence of acridinium ester. In addition, even if a small part of the acridinium ester has the background fluorescence, the acridinium ester will not excite the quantum dot to emit the fluorescence on a basis of the fluorescence resonance energy transfer since there is no the stem-loop structure. Therefore, the embodiments of the present application can minimize the impact of the background fluorescence of acridinium ester on the measurement result, thereby reducing the measurement error.

In some embodiments, the fluorescence emitted by the quantum dot can be obtained via an optical filter and a photomultiplier tube (PMT). Exemplarily, if the fluorescence emitted by the quantum dot is 605nm, then the optical filter only allows the light at a wavelength of 605nm to pass therethrough.

In some embodiments, the antioxidant may be selected from any one or more of cannabidiol, vitamin C, vitamin E, tea polyphenols, and glutathione. The oxidant may include an alkaline solution of hydrogen peroxide. After removing the antioxidant, the added oxidant may cause the first fluorescent group acridinium ester to emit the fluorescence.

Embodiments of the present application use the immune reaction to enable the antibody to recognize the small molecule substance, thereby drawing the spacing distance between single-stranded DNAs that can be complementarily paired nearer, which in turn initiates DNA assembly, triggers cascade DNA assembly and causes production of the fluorescence resonance energy transfer effect between two fluorescent molecules to generate the detection signal.

Each embodiment of the present application uses acridinium ester (AE) as the fluorescence energy donor (donor), the fluorescent quantum dot (QDs) as the fluorescence energy acceptor (acceptor), and is based on the chemiluminescence resonance energy transfer system. The fluorescence signal amplification is achieved via immune reaction and DNA self-assembly, and the detection of the content of small molecule substance is converted into the detection of fluorescence signal intensity. The methods of the embodiments of the present application all can be performed in a homogeneous phase. Compared with the traditional fluorescence resonance energy transfer analysis method, the detection method of embodiments of the present application is simple and fast, without requiring expensive laser. In the embodiments of the present application, the second fluorescence is used as the detection fluorescence, which reduces the background interference of the first fluorescence. The embodiments of the present application use the immune reaction to enrich the small molecule substance, which has good selectivity for the small molecule substance and high detection sensitivity.

### [A method for detecting a small molecule substance]

As shown in FIG. 2, some embodiments of the present application provide a detection method for the small molecule substance, and the detection method may use the above-mentioned reagent combination. The features regarding the reagent combination described above may be incorporated into this section. The detection method of the above embodiment includes the following steps:
(1) Adding the first conjugate, the second conjugate, the third conjugate and the oxidation inhibiting reagent to the solution to be tested, and performing mixing to form the sample to be tested. The first conjugate is formed at least by coupling a first nucleic acid molecule to an antibody. The second conjugate is formed at least by sequentially coupling a substrate protein conjugate, a second nucleic acid molecule and a second fluorescent group. The third conjugate is formed at least by coupling a first fluorescent group to a third nucleic acid molecule. In the condition that the solution to be tested contains no the small molecule substance or a small amount of the small molecule substance, the antibody and the substrate protein conjugate form an immune complex, and the first nucleic acid molecule, the second nucleic acid molecule and the third nucleic acid molecule form a stem-loop structure, so that the first fluorescent group and the second fluorescent group are located on the same side of the stem-loop structure. In the condition that the solution to be tested contains a large number of the small molecule substance, the antibody specifically binds to the small molecule substance and loses its binding to the small molecule substrate in the substrate protein conjugate, and the immune complex and stem-loop structure cannot be formed.
(2) Removing the oxidation inhibiting reagent from the sample to be tested, adding an oxidant used to oxidize the first fluorescent group to emit the first fluorescence, and collecting the second fluorescence according to the maximum emission wavelength of the second fluorescent group. The oxidation inhibiting reagent contains the antioxidant for inhibiting the first fluorescent group from being oxidized by the oxidizing substance in the solution to be tested. Since the first fluorescent group does not emit the first fluorescence in the presence of the antioxidant, the antioxidant needs to be removed in advance before adding the oxidant. In addition, the first fluorescent group will be subjected to the antioxidant protection by the antioxidant before being oxidized, thereby also reducing the background fluorescence emitted by itself due to the influence of other factors.
(3) In a case that the second fluorescence is collected, obtaining the content of the small molecule substance according to the intensity of the second fluorescence and the one-to-one correspondence between the intensity of the fluorescence and the content of the small molecule substance; in a case that the second fluorescence is not collected, it means that the solution to be tested contains an excess of the small molecule substance, resulting in the complete inability of the small molecule substrate to bind to the antibody. Of course, the latter is an extreme case. Therefore, various embodiments of the present application can still obtain the concentration of the small molecule substance in the solution to be tested according to the intensity of the second fluorescence.

In this step, the first fluorescent group will produce the first fluorescence after being oxidized. In order to avoid the impact of the first fluorescence on the detection result, the optical filter is used to filter out the first fluorescence. The optical filter only allows the transmission of the second fluorescence emitted by the second fluorescent group. At this time, the content of the small molecule substance is obtained according to the intensity of the second fluorescence. If the relative intensity of the second fluorescence is 0, it means that the solution to be tested contains an excess amount of the small molecule substance. If the relative intensity of the second fluorescence is not 0, then the content of the small molecule substance in the solution to be tested is obtained according to the functional relationship between the relative intensity of the second fluorescence and the content of the small molecule substance. The relative intensity refers to the remaining intensity value of the fluorescence after excluding the background fluorescence intensity of the control group from the intensity of the second fluorescence signal.

In some embodiments, the working concentration of the first conjugate in the sample to be tested range from 1 nM to 20 nM. The working concentration refers to the concentration of each reagent during actual detection, that is, the final concentration of each reagent in the sample to be tested. The working concentration is not equal to the storage concentration of each reagent in the kit for detecting the small molecule substance. In some embodiments, the working concentration of the second conjugate in the sample to be tested range from 1 nM to 20 nM. In some embodiments, the working concentration of the third conjugate in the sample to be tested range from 0.05 nM to 0.2 nM. In some embodiments, the working concentration of the oxidation inhibiting reagent in the sample to be tested range from 15 µg/ml to 25 µg/ml.

In some embodiments, the blood sample of the solution to be tested is derived from a whole blood sample, a serum sample or a plasma sample.

In some embodiments, the mixing duration time is 5 minutes to 10 minutes. In some embodiments, the temperature of mixing is 36 to 37 degrees.

In some embodiments, the volume of the oxidant is 200 µL. The oxidant is the alkaline hydrogen peroxide solution with a pH value of 8.0. The alkaline hydrogen peroxide solution is prepared by dissolving hydrogen peroxide in TBS buffer, where the final concentration of hydrogen peroxide is 0.1M and the final concentration of TBS is 10 mM.

In some embodiments, the small molecule substance includes triiodothyronine, tetraiodothyronine, or progesterone. However, in other embodiments, the small molecule substance is not limited to the above several kinds, as long as the antibody of the present application can specifically bind to the small molecule substance and form the stable immune complex. One antibody can only bind to one small molecule substance or one small molecule substrate. When the antibody is bound to the small molecule substrate, the relatively stable immune complex will be formed. The immune complex will draw the distance between the first nucleic acid molecule, the second nucleic acid molecule and the third nucleic acid molecule closer to form the stem-loop structure. The first fluorescent group and the second fluorescent group are located on the same side of the stem-loop structure. The first fluorescent group can then excite the second fluorescent group to produce the second fluorescence. Since the small molecule substance or the small molecule substrate each has the one-to-one correspondence with the antibody and the second fluorescent group, and the strongest fluorescence degree of the second fluorescent group is known, then the number of the small molecule substance is linearly related to the fluorescence quenching degree of the second fluorescent group, on a basis of which the relationship between the number of the small molecule substance and the remaining fluorescence intensity can also be obtained. The content of the small molecule substance may be calculated according to the functional relationship between the intensity of the second fluorescence and the content of the small molecule substance contained in the standard curve and the obtained fluorescence intensity of the second fluorescent group.

In some embodiments, the specific detection steps are as follows: mixing the detection reagents (the first conjugate, the second conjugate, the third conjugate and the oxidation inhibiting reagent) with the blood sample or the whole blood sample or the serum sample or the plasma sample to be tested containing the small molecule substance, and incubating and reacting the mixture on the chemiluminescence detector at 37°C for 5-10 minutes, adding the chemiluminescence substrate hydrogen peroxide, sodium hydroxide and TBS buffer as the oxidant, and collecting the generated chemiluminescence fluorescent signal via the PMT detection module in the chemiluminescence detector. The chemiluminescence detector automatically calls the standard curve and reports the concentration of the small molecule substance in the sample to be tested according to the functional relationship between the intensity of the fluorescence and the content of the small molecule substance contained in the standard curve.

### [A kit for detecting a small molecule substance]

Some embodiments of the present application provide a kit for detecting a small molecule substance, which performs the detection using the above detection method. The small molecule detection kit includes: the first storage tube, the second storage tube, the third storage tube, the fourth storage tube, the fourth storage tube and the fifth storage tube.

The first storage tube stores the conjugate of the first nucleic acid molecule and the antibody. The first nucleic acid molecule contains 55 bases, and the 3' end is modified with an NH2C7 group. The 3'C7 amino-modified primer was purchased from Genscript Biotechnology Co., Ltd. The NH2C7 group is covalently connected to the amino group of the antibody via the first coupling agent suberate bis(sulfosuccinimidyl) sodium salt (BS3), thereby forming the conjugate of the first nucleic acid molecule and the antibody.

The second storage tube stores the conjugate of the substrate protein conjugate, the second nucleic acid molecule and the second fluorescent group. The antibody and the substrate protein conjugate form the immune complex in the absence or presence of the small amount of the small molecule substance. The second nucleic acid molecule contains 53 bases, with a thiol group modified at the 3' end and an NH2C6 group modified at the 5' end. The thiol modification reagent 3' SH C6 and 5' Aminolinker (C6) modification primer were purchased from Genscript Biotechnology Co., Ltd. The 5' end modification is added to the 5' sugar ring in the form of amine phosphite via a β-cyanoethyl chemical reaction in the last step of the synthesis cycle, instead of being added to the last base. The 3'-terminal thiol group is covalently connected to the amino group on the surface of the amino-modified quantum dot QDs via the third coupling agent 4-(N-maleimidomethyl)cyclohexanecarboxylic acid N-hydroxysuccinimide ester (SMCC). In some embodiments, the model of the amino-modified water-soluble quantum dot may be amino water soluble quantum dot (PEG)-605, which is composed of CdSe/ZnS and is purchased from Xi'an Qiyue Biotechnology Co., Ltd. (PEG)-605 has a maximum absorption peak wavelength of 470 nm, a maximum emission wavelength of 605 nm, and a particle size of 4.1nm. The amino group of the amino-modified quantum dot is coupled with the thiol group at the 3' end of the second nucleic acid molecule to form the conjugate of the second nucleic acid molecule and the quantum dot. The NH2C6 group at the 5' end of the second nucleic acid molecule is covalently bonded to the amino group on the substrate protein conjugate via the second coupling agent BS3, forming the conjugate between the substrate protein conjugate, the second nucleic acid molecule and the second fluorescent group.

The third storage tube stores the conjugate of the first fluorescent group and the third nucleic acid molecule; The first nucleic acid molecule, the second nucleic acid molecule and the third nucleic acid molecule can form the stem-loop structure in the condition that the immune complex is formed; The first fluorescent group and the second fluorescent group can be located on the same side of the stem-loop structure. The third nucleic acid molecule contains 22 bases, and the 5' end is modified with an NH2C6 group. The modified primer 5' Aminolinker (C6) is added to the sugar ring at the 5' end of the third nucleic acid molecule via a β-cyanoethyl chemical reaction in the form of amine phosphite in the last step of the synthesis cycle, instead of being added to the last base. The modified primer was purchased from Genscript Biotechnology Co., Ltd. The conjugate of the first fluorescent group and the third nucleic acid molecule was formed by adding acridinium ester (NSP-DMAE-NHS). The first fluorescent group acridinium ester was purchased from Suzhou Yake Technology Co., Ltd., CAS number is 194357-64-7. The bases at 3 to 10 base sites of the third nucleic acid molecule starting from the 5' end are completely complementary to the bases at 3 to 10 base sites of the second nucleic acid molecule starting from the 3' end (i.e., the 44th to 51st base sites starting from the 5' end). The bases at 5 to 12 base sites of the third nucleic acid molecule starting from the 3' end (i.e., the 11th to 18th base sites starting from the 5' end) are completely complementary to the bases at 3 to 10 base sites of the first nucleic acid molecule starting from the 5' end, as shown in FIG. 1.

The fourth storage tube stores the antioxidant capable of inhibiting the first fluorescent group from being oxidized. The antioxidant may bind to the surface of the carrier molecule. In some embodiments, the carrier molecule may be graphene oxide.

The fifth storage tube stores the oxidant capable of oxidizing the first fluorescent group to emit the first fluorescence.
the detection principle of the small molecule substance in the above embodiment is as follows:

The third nucleic acid molecule has respectively 8 bases that are complementarily paired to the first nucleic acid molecule and the second nucleic acid molecule, where the natural base pairs G and C are replaced with the unnatural base pairs ᵢₛₒG and ᵢₛₒC.

In the presence of the excess amount of the small molecule substance, the stem-loop structure will not be formed. The third nucleic acid molecule is adsorbed on the surface of graphene oxide (GO) via π-π stacking function, and the acridinium ester (AE) terminally labelled thereof can't be oxidized for luminescence due to the presence of the oxidant. Even if a small part of acridinium ester produces the background fluorescence, the chemiluminescence of acridinium ester (430nm wavelength) cannot be detected by PMT due to the optical filter of the instrument (only allowing 605nm light to pass therethrough).

In the absence of the small molecule substance or the presence of a small amount of the small molecule substance, the antibody and substrate protein conjugate can form the immune complex. Since the antibody is coupled to the first nucleic acid molecule, and the substrate protein conjugate is coupled to the second nucleic acid molecule, the immune complex brings the first nucleic acid molecule and the second nucleic acid molecule close enough to form an ortho-complex, and can hybridize with the third nucleic acid molecule, so that the first nucleic acid molecule, the second nucleic acid molecule and the third nucleic acid molecule form the stem-loop structure, and the first fluorescent group and the second fluorescent group are located on the same side of the stem-loop structure. The complex formed by the antibody and DNA is basically not adsorbed by the carrier molecule graphene oxide, so the antioxidant coupled to the carrier molecule will basically not affect the luminescence of acridinium ester. In the condition that the antioxidant is removed, the added oxidant can oxidize the first fluorescent group and cause it to emit the first fluorescence. The first fluorescence excites the second fluorescent group to emit the second fluorescence (for example, 605nm) on a basis of the fluorescence resonance energy transfer, in order to obtain the content of the small molecule substance on a basis of the intensity of the second fluorescence.

### [A system for detecting a small molecule substance]

The present application provides a system for detecting a small molecule substance, which includes: a reaction vessel, a microsyringe pump, an optical filter and a calculation module.
the reaction vessel has an accommodation chamber capable of accommodating the solution to be tested.

The microinjection pump is communicated with the accommodation chamber of the reaction vessel through the injection pipeline, and injects the mixture of the first conjugate, the second conjugate, the third conjugate and the oxidation inhibiting reagent into the accommodation chamber; The first conjugate is formed at least by coupling the first nucleic acid molecule to the antibody, the second conjugate is formed at least by sequentially coupling a substrate protein conjugate, a second nucleic acid molecule and a second fluorescent group, and the third conjugate is formed at least by coupling the first fluorescent group to the third nucleic acid molecule, and the oxidation inhibiting reagent contains the antioxidant that inhibits the first fluorescent group from being oxidized to emit the first fluorescence.

The optical filter is disposed on the exit light path of the first fluorescence and allows the transmission of the second fluorescence that has the same as the maximum emission wavelength of the second fluorescence group.

The light signal detection module is disposed on the downstream side of the optical filter in the exit light path of the fluorescence signal, and acquires the second fluorescence transmitted through the optical filter.

The calculation module converts the second fluorescence into the digital signal and obtains the content of the small molecule substance in the solution to be tested according to the functional relationship between the intensity of the fluorescence and the content of the small molecule substance. The standard curve contains a standard equation, which contains the one-to-one correspondence between the digital signal and the content of the small molecule substance.

The embodiments of the present application provide a simple, fast, and sensitive homogeneous chemiluminescence immunoassay protein detection method via the dual quenching mechanism of the graphene oxide coupled antioxidant and the optical filter in combination with the chemiluminescence resonance energy transfer and immunoassay technology. Compared with the existing immunoassay method, some embodiments of the present application have the following characteristics:
(1) Some embodiments of the present application are the homogeneous immunoassay method, which is simple to operate and greatly shorten the turnaround time (TAT) of the clinical test samples. There is no need for centrifugation of the blood samples and the whole blood can be loaded and a test report is issued in about 5 minutes.
(2) In some embodiments of the present application, the acridinium ester involves oxidative luminescence, and the quantum dot involves excited luminescence, which can realize self-excited luminescence between acridinium ester and the quantum dot without the need to set up the complex external excitation luminescent system. The system can effectively reduce the complexity and cost of the measuring instrument. As the requirements for supporting the testing equipment are reduced, the modules are reduced, the cost is reduced, and the failure rate is also greatly reduced, the automatic testing or the miniaturized portable point-of-care testing (POCT) can be achieved.
(3) The acridinium ester-quantum dot luminescence system of some embodiments of the present application introduces a dual quenching mechanism (graphene oxide-reducing agent and an optical filter), with lower background fluorescence and higher detection sensitivity, and is suitable for higher sensitivity detection.
(4) Some embodiments of the present application induce the switching of the graphene oxide-antioxidant quenching mechanism via the immune response, coupled with the introduction of the external optical filter, and the switching efficiency reaches 100%, thereby enabling the quantum dot to emit light combined with the chemiluminescence resonance energy transfer effect, without the need for separation and cleaning steps.
(5) The DNA molecules in some embodiments of the present application contain non-natural base pairs (ᵢₛₒG and ᵢₛₒC) to avoid non-specific binding to the nucleic acid in the sample.

The technology of the present application will be further described below in conjunction with each of Preparation Examples and each of Examples.

Preparation Example 1 Preparation of the conjugate of the second nucleic acid molecule and the second fluorescent group (quantum dot)

The present preparation example provides a method for preparing the conjugate of the second nucleic acid molecule and the quantum dot, which includes the following steps:
1. Formulation of SMCC solution: 10mg of 4-(N-maleimidomethyl)cyclohexanecarboxylic acid N-hydroxysuccinimide ester (SMCC) is weighed and dissolved in 1mL in DMF. SMCC was purchased from Shanghai Aladdin Bio-Chem Technology Co., LTD., the product number is N159712, and the CAS number is 64987-85-5.
2. Formulation of the second nucleic acid molecule solution: 10 µM of the second nucleic acid molecule was taken and added to 1 mL of purified water to dissolve it.
3. Preparation of the conjugate of the second nucleic acid molecule and the quantum dot: 100 µL of the second nucleic acid molecule solution was taken in the EP tube, 200 µL of QDs (purchased from Xi'an Qiyue Biotechnology Co., Ltd., the model number is amino water-soluble quantum dot (PEG)-605, with the concentration of 8 µM) was added, 3 µL of SMCC solution was added, and mixed evenly, incubated at 37°C for 30 minutes. QDs were purchased from Xi'an Qiyue Biotechnology Co., Ltd., with a model name being amino water-soluble quantum dot (PEG)-605, and the concentration being 8 µM.
4. Dialysis: the conjugate of the second nucleic acid molecule and the quantum dot which have finished coupling was aspirated from the EP tube and added to the dialysis bag (5kd specification), the dialysis bag was tied and put into a beaker loaded with 2-3L TE solution (10mM Tris, 1mM EDTA, pH=8.0) for dialysis. The dialysis bag was soaked in advance before dialysis. The dialysate was changed once every 2-3 hours, and dialyzed three times in total. After dialysis, the liquid in the dialysis bag was collected into a centrifuge tube and stored at 2-8°C for later use.

Preparation Example 2 Preparation of the conjugate of the first fluorescent group (acridinium ester) and the third nucleic acid molecule

The present preparation example provides a method for preparing the conjugate of acridinium ester (AE) and the third nucleic acid molecule, which includes the following steps:
1. Formulation of the third nucleic acid molecule solution: 20 µM of the third nucleic acid molecule was taken and added to 1 mL of purified water to dissolve it.
2. Formulation of NHS-AE solution: 4 mg of acridinium ester (NSP-DMAE-NHS) was weighed and dissolved in 1 mL of purified water. Acridinium ester was purchased from Suzhou Yake Technology Co., Ltd., with CAS number 194357-64-7.
3. Coupling: 10 µL of the third nucleic acid molecule solution was added per 1 mg of NHS-AE solution, mixed evenly in an EP tube, and incubated at 37°C for 30 minutes.
4. Dialysis: the coupled product was aspirated from the EP tube, added to the dialysis bag (5kd specification), the dialysis bag was tied and put into a beaker containing 2-3L TE solution (10mM Tris, 1mM EDTA, PH=8.0) for dialysis (the dialysis bag was soaked in advance), the dialysate was changed once every 2-3 hours, and dialyzed three times in total. After dialysis, the liquid in the dialysis bag was collected into the centrifuge tube and stored at 2-8°C for later use.

### Preparation Example 3 Preparation of conjugate of the first nucleic acid molecule and the antibody

The present preparation example provides a method for preparing the conjugate of the first nucleic acid molecule and the antibody, which includes the following steps:
1. Formulation of BS3 solution: 10 mg of BS3 (BS3) was weighed and dissolved in 1 mL of purified water. BS3 was purchased from Shanghai Aladdin Bio-Chem Technology Co., LTD., with Item Number S304724.
2. Activation: the aliquoted antibody was taken out, thawed, and centrifuged homogenously. 3 µL of BS3 solution was added per 1 mg of antibody, 6.5 µL of the first nucleic acid molecule was added, mixed evenly in an EP tube, and incubated at 37°C for 30 minutes.
3. Dialysis: the conjugate of the first nucleic acid molecule and the antibody was aspirated from the EP tube, added to the dialysis bag (100kd specification), the dialysis bag was tied and put into a beaker containing 2-3L PBS solution for performing dialysis (the dialysis bag was soaked in advance), the dialysate was changed once every 2-3 hours, and dialyzed three times in total. After dialysis, the liquid in the dialysis bag was collected into the centrifuge tube and stored at 2-8°C for later use.

Preparation Example 4 Preparation of the conjugate of the substrate protein conjugate, the second nucleic acid molecule and the second fluorescent group

The present preparation example provides a method for preparing the conjugate of the substrate protein conjugate, the second nucleic acid molecule, and the second fluorescent group, which includes the following steps:
1. Formulation of BS3 solution: 10 mg of suberate bis(sulfosuccinimidyl) sodium salt (BS3) was weighed and dissolved in 1 mL of purified water. BS3 was purchased from Shanghai Aladdin Bio-Chem Technology Co., LTD.
2. Activation: the aliquoted substrate protein conjugate (the conjugate of the small molecule substrate and the scaffold protein) was taken out, thawed and centrifuged homogeneously. 3 µL of BS3 solution was added per 1 mg of the substrate protein conjugate, 6.5 µL of the conjugate of the second nucleic acid molecule and the quantum dot was added, mixed evenly in an EP tube and incubated at 37°C for 30 minutes.
3. Dialysis: the conjugate of the substrate protein conjugate, the second nucleic acid molecule and the second fluorescent group was aspirated from the EP tube, added to the dialysis bag (100kd specification), the dialysis bag was tied and put into a beaker containing 2-3L PBS solution for performing dialysis (the dialysis bag was soaked in advance), the dialysate was replaced once every 2-3 hours, and dialyzed three times in total. After dialysis, the liquid in the dialysis bag was collected into a centrifuge tube and stored at 2-8°C for later use.

### Example 1

The present Example uses a homogeneous immunoassay method based on graphene oxide-antioxidant quenching and acridinium ester chemiluminescence to detect free triiodothyronine (FT3) in serum. Among them, the antibody was purchased from Bioventix with the clone number 17c6r; The substrate protein conjugate (also known as the scaffold small molecule) were purchased from Nanjing Dawn Biotechnology, with the clone number T3-KLH. The antibody and the substrate protein conjugate are coupled to the corresponding DNA molecule via the above preparation example. The specific detection method includes the following steps:
1. Formulating the detection solution: The conjugate of the first nucleic acid molecule and antibody (DNA1-antibody conjugate), the substrate protein conjugate, the conjugate of the second nucleic acid molecule and the quantum dot (scaffold protein-DNA2-QDs conjugate), the conjugate of acridine and the third nucleic acid molecule, the antioxidant-modified graphene oxide (GO-AOD) were mixed so that their final concentrations were 10 nM, 10 nM, 0.15 µM, and 20 µg/ml, respectively.
2. 50 µL of the calibration solution at the different concentrations or the serum samples containing triiodothyronine (serum T3) was mixed with 200 µL of the detection solution, and incubated at 37°C for 5-10 minutes.
3. After incubation, 200 µL of the chemiluminescent substrate was added via the HSCL-10000 chemiluminescence instrument. The chemiluminescent substrate includes: 10mM TBS buffer and 0.1M alkali solution of hydrogen peroxide at pH=8.0, and the chemiluminescence signal of the solution was detected immediately via a photomultiplier tube (PMT) with a detection time of 3 seconds. According to the recorded chemiluminescence values (RLU), the calibration curve of triiodothyronine and the concentration of serum FT3 in the serum sample to be tested were obtained.

After testing 40 clinical samples, the error between the FT3 detection value of the present Example and the Roche test value was 1.82%, indicating that the detection method of the present Example has higher accuracy. The test results are shown in Table 1 below. The Roche test refers to the Roche FT3 kit.

**Table 1 is a comparison table of the detection values of the present Example and the Roche detection method.**

| FT3 Sample | Roche (pmol/L) | The Present Example (pmol/L) | Deviation | FT3 Sample | Roche (pmol/L) | The Present Example (pmol/L) | Deviation |
|---|---|---|---|---|---|---|---|
| 1 | 0.77 | 0.85 | 10.39% | 21 | 35.63 | 37.87 | 6.29% |
| 2 | 0.73 | 0.8 | 9.59% | 22 | 20.18 | 17.66 | -12.49% |
| 3 | 1 | 1.11 | 11.00% | 23 | 23. 51 | 20.37 | -13.36% |
| 4 | 0.65 | 0.71 | 9.23% | 24 | 40.75 | 43.72 | 7.29% |
| 5 | 0.86 | 0.8 | -6.98% | 25 | 27.93 | 31.63 | 13.25% |
| 6 | 0.7 | 0.73 | 4.29% | 26 | 45.03 | 41.87 | -7.02% |
| 7 | 0.72 | 0.66 | -8.33% | 27 | 19.12 | 20.03 | 4.76% |
| 8 | 0.66 | 0.66 | 0.00% | 28 | 32.84 | 29.02 | -11.63% |
| 9 | 0.99 | 0.85 | -14.14% | 29 | 37.21 | 32. 5 | -12.66% |
| 10 | 0.95 | 1.04 | 9.47% | 30 | 17.01 | 17.24 | 1.35% |
| 11 | 1.57 | 1.46 | -7.01% | 31 | 34.01 | 36.93 | 8.59% |
| 12 | 4.62 | 4.76 | 3.03% | 32 | 46.66 | 45.49 | -2.51% |
| 13 | 9.06 | 9.95 | 9.82% | 33 | 19.35 | 20.79 | 7.44% |
| 14 | 3.56 | 3.42 | -3.93% | 34 | 36.23 | 39.7 | 9.58% |
| 15 | 8.19 | 9.39 | 14.65% | 35 | 11.57 | 10.12 | -12.53% |
| 16 | 1.72 | 1.67 | -2.91% | 36 | 39.62 | 43.62 | 10.10% |
| 17 | 8.76 | 9.02 | 2.97% | 37 | 26.89 | 30.88 | 14.84% |
| 18 | 2.71 | 2.92 | 7.75% | 38 | 32.48 | 32.92 | 1.35% |
| 19 | 1.61 | 1.79 | 11.18% | 39 | 12.01 | 12.12 | 0.92% |
| 20 | 3.96 | 3.69 | -6.82% | 40 | 18.25 | 19.37 | 6.14% |
| Total Error | | | | | | | 1.82% |

### Example 2

The present Example uses a homogeneous immunoassay method on a basis of graphene oxide-antioxidant quenching and acridinium ester chemiluminescence to detect tetraiodothyronine (serum T4) in serum. Among them, the antibody was purchased from Nanjing Oukai Biotechnology Co., Ltd., with the clone number K88a6, and the substrate protein conjugate (also known as scaffold small molecule) was purchased from Shanghai Hanzun Biotechnology, with the clone number T4-BSA-HT-020561. The antibody and the substrate protein conjugate are coupled to the corresponding DNA via the above preparation example. The specific detection method includes the following steps:
1. Formulating the detection solution: the conjugate of the first nucleic acid molecule and the antibody, the substrate protein conjugate, the conjugate of the second nucleic acid molecule and the quantum dot, the conjugate of the acridinium ester and the third nucleic acid molecule, and the antioxidant-modified graphene oxide were mixed so that their final concentrations are 10 nM, 10 nM, 0.15 µM and 20 µg/ml respectively.
2. 50 µL of calibration solutions at the different concentrations or the serum samples containing tetraiodothyronine were mixed with 200 µL of detection solution, and incubated at 37°C for 5-10 minutes.
3. After incubation, 200 µL of the chemiluminescent substrate was added via the HSCL-10000 chemiluminescence instrument. The chemiluminescent substrate includes: 10mM TBS buffer, 0.1M alkaline solution of hydrogen peroxide at pH=8.0. The chemiluminescence signal of the solution was detected immediately via a photomultiplier tube (PMT) with a detection time of 3 seconds. According to the recorded chemiluminescence values (RLU), the calibration curve of serum FT4 and the concentration of serum T4 in the serum sample to be tested were obtained.

After multiple tests, the detection limit of the serum FT4 in present Example is 0.5-100 pmol/L. After testing 40 clinical samples, the error between the FT4 detection value of the present Example and the Roche test value was -1.66%, indicating that the detection method of the present Example has higher accuracy. The test results are shown in Table 2 below. The Roche test refers to the Roche FT4 kit.

**Table 2 is a comparison table of the detection values of the present Example and the Roche detection method.**

| FT4 Sample | Roche (pmol/L) | The Present Example (pmol/L) | Deviation | FT4 Sample | Roche (pmol/L) | The Present Example (pmol/L) | Deviation |
|---|---|---|---|---|---|---|---|
| 1 | 0.89 | 0.95 | 6.74% | 21 | 46.06 | 45.59 | -1.02% |
| 2 | 0.92 | 0.94 | 2.17% | 22 | 80.33 | 90.96 | 13.23% |
| 3 | 0.7 | 0.62 | -11.43% | 23 | 18.19 | 16.17 | -11.11% |
| 4 | 0.7 | 0.61 | -12.86% | 24 | 45.26 | 39.49 | -12.75% |
| 5 | 0.85 | 0.96 | 12.94% | 25 | 74**.**52 | 70.75 | -5.06% |
| 6 | 0.76 | 0.76 | 0.00% | 26 | 64 | 63.77 | -0.36% |
| 7 | 0.67 | 0.72 | 7*.*46% | 27 | 70.81 | 72.78 | 2.78% |
| 8 | 0.63 | 0.56 | -11.11% | 28 | 29.99 | 33.12 | 10.44% |
| 9 | 0.55 | 0.52 | -5.45% | 29 | 78.47 | 74.78 | -4.70% |
| 10 | 0.54 | 0.56 | 3.70% | 30 | 70.55 | 60.31 | -14.51% |
| 11 | 3.13 | 4.5 | -12.28% | 31 | 54**.**28 | 49.59 | -8.64% |
| 12 | 4.44 | 4.59 | 3.38% | 32 | 53.56 | 59.4 | 10.90% |
| 13 | 1.67 | 1.48 | -11.38% | 33 | 75.16 | 76.57 | 1.88% |
| 14 | 3.76 | 3.24 | -13.83% | 34 | 17.74 | 15.27 | -13.92% |
| 15 | 5.84 | 5.75 | -1.54% | 35 | 11.79 | 11.78 | -0.08% |
| 16 | 6.41 | 6.74 | 5.15% | 36 | 68.72 | 76.2 | 10**.**88% |
| 17 | 5.37 | 5.74 | 6.89% | 37 | 31.02 | 35.4 | 14.12% |
| 18 | 3.07 | 2.83 | -7.82% | 38 | 96.36 | 86.4 | -10.34% |
| 19 | 1.22 | 1.05 | -13.93% | 39 | 84.39 | 88.26 | 4.59% |
| 20 | 4.74 | 5.37 | 13.29% | 40 | 37.35 | 32**.**58 | -12.77% |
| Total Error | | | | | | | -1.66% |

### Example 3

The present Example uses a homogeneous immunoassay method on a basis of graphene oxide-antioxidant quenching and acridinium ester chemiluminescence to detect progesterone (Prog) in serum. Among them, the antibody was purchased from Biospacific, with the clone number PPOGESTERONE MAB, PURE/A25050; the substrate protein conjugate (also known as scaffold small molecule) was purchased from Biospacific, with the clone number PPOGESTERONE-11-BSA/V56050. The antibody and the substrate protein conjugate are coupled to the corresponding DNA via the above preparation example. The specific detection method includes the following steps:
1. Formulating the detection solution: the conjugate of the first nucleic acid molecule and the antibody, the substrate protein conjugate, the conjugate of the second nucleic acid molecule and the quantum dot, the conjugate of the acridinium ester and the third nucleic acid molecule, and the antioxidant-modified graphene oxide were mixed so that their final concentrations are 10 nM, 10 nM, 0.15 µM and 20 µg/ml respectively.
2. 50 µL of the calibration solutions at different concentrations or the serum samples containing progesterone were mixed with 200 µL of the reagent solution, and incubated at 37°C for 5-10 minutes.
3. After incubation, 200 µL of the chemiluminescent substrate was added via the HSCL-10000 chemiluminescence instrument. The chemiluminescent substrate includes: 10mM TBS buffer, 0.1M alkaline solution of hydrogen peroxide at pH=8.0. The chemiluminescence signal of the solution was detected immediately via a photomultiplier tube (PMT) with a detection time of 3 seconds. According to the recorded chemiluminescence values (RLU), the calibration curve of Prog and the concentration of Prog in the serum sample to be tested were obtained.

After multiple tests, the detection limit of Prog in the present Example is 0.05-60ng/mL. After testing 40 clinical samples, the error between the Prog detection value of the present Example and the Roche test value was 2.63%, indicating that the detection method of the present Example has higher accuracy. The test results are shown in Table 3 below. The Roche test refers to the Roche Prog test kit.

**Table 3 is a comparison table of the detection values of the present Example and the Roche detection method.**

| Prog Sample | Roche (ng/mL) | The Present Example (ng/mL) | Deviation | Prog Sample | Roche (ng/mL) | The Present Example (ng/mL) | Deviation |
|---|---|---|---|---|---|---|---|
| 1 | 1.05 | 1 | -4.76% | 21 | 10.19 | 11.31 | 10.99% |
| 2 | 0.31 | 0.32 | 3.23% | 22 | 47.97 | 47.22 | -1**.**56% |
| 3 | 0.66 | 0.61 | -7.58% | 23 | 36.69 | 38.45 | 4.80% |
| 4 | 1.93 | 2.2 | 13.99% | 24 | 48.45 | 47.66 | -1.63% |
| 5 | 1.81 | 1.71 | -5.52% | 25 | 32.79 | 37.33 | 13.85% |
| 6 | 0.57 | 0.57 | 0.00% | 26 | 21.23 | 21.96 | 3.44% |
| 7 | 0.05 | 0.06 | 20.00% | 27 | 54.38 | 51.74 | -4.85% |
| 8 | 0.59 | 0.58 | -1.69% | 28 | 16. 58 | 16.02 | -3.38% |
| 9 | 1.86 | 2.07 | 11.29% | 29 | 30.95 | 32.87 | 6.20% |
| 10 | 1.96 | 1.79 | -8.67% | 30 | 52.25 | 45.99 | -11.98% |
| 11 | 9.31 | 9.29 | -0.21% | 31 | 33.12 | 32.35 | -2.32% |
| 12 | 8.49 | 7.93 | -6.60% | 32 | 39.8 | 45.18 | 13.52% |
| 13 | 5.77 | 6.15 | 6.59% | 33 | 51.18 | 53.2 | 3.95% |
| 14 | 6.37 | 6.56 | 2.98% | 34 | 31.11 | 33.69 | 8.29% |
| 15 | 4.81 | 4.91 | 2.08% | 35 | 58.27 | 55.99 | -3.91% |
| 16 | 8.23 | 7.74 | -5.95% | 36 | 48.2 | 52.83 | 9.61% |
| 17 | 5.93 | 6.71 | 13.15% | 37 | 29.24 | 27.67 | -5.37% |
| 18 | 6.92 | 7.61 | 9.97% | 38 | 35.55 | 39.53 | 11.20% |
| 19 | 3.01 | 3**.**3 | 9.63% | 39 | 50.21 | 52.39 | 4.34% |
| 20 | 4.13 | 4.56 | 10.41% | 40 | 19.52 | 17.08 | -12.50% |
| Total Error | | | | | | | 2.63% |

The present application has been described by the above-mentioned relevant embodiments, but the above-mentioned embodiments are only examples of implementing the present application. It must be noted that the disclosed embodiments do not limit the scope of the present application. On the contrary, modifications and equivalent arrangements included in the spirit and scope of the claims are included in the scope of the present application.

## Claims

1. A reagent combination for detecting a small molecule substance, comprising:
a first conjugate formed at least by coupling a first nucleic acid molecule to an antibody; wherein the first nucleic acid molecule comprises a first hybridization zone and a second hybridization zone; the antibody is capable of specifically binding to the small molecule substance;
a second conjugate formed at least by sequentially coupling a substrate protein conjugate, a second nucleic acid molecule and a second fluorescent group; wherein the substrate protein conjugate is at least formed by coupling a small molecule substrate to a scaffold protein, the small molecule substrate is the same as the small molecule substance; the second nucleic acid molecule has a third hybridization zone and a fourth hybridization zone, and the third hybridization zone is complementary to the second hybridization zone;
a third conjugate formed at least by coupling a first fluorescent group to a third nucleic acid molecule; wherein the third nucleic acid molecule comprises a fifth hybridization zone and a sixth hybridization zone, the fifth hybridization zone is complementary to the fourth hybridization zone; the sixth hybridization zone is complementary to the first hybridization zone; the first fluorescent group emits a first fluorescence in a condition that the antibody binds to the small molecule substrate in the substrate protein conjugate and is capable of being oxidized by an oxidant, and the first fluorescence excites the second fluorescent group to emit a second fluorescence in a condition that the first nucleic acid molecule, the second nucleic acid molecule and the third nucleic acid molecule are paired with each other to obtain a content of the small molecule substance according to an intensity of the second fluorescence and a one-to-one correspondence between the intensity of the fluorescence and the content of the small molecule substance; and
an oxidation inhibiting reagent comprising: an antioxidant for inhibiting the first fluorescent group from being oxidized to emit the first fluorescence.

2. The reagent combination for detecting the small molecule substance as claimed in claim 1, wherein the antibody specifically binds to the small molecule substrate in the small molecule substrate conjugate in the condition that a solution to be tested does not comprise or comprises a small amount of the small molecule substance, such that the antibody and the substrate protein conjugate form an immune complex, and the first nucleic acid molecule, the second nucleic acid molecule, and the third nucleic acid molecule are complementarily paired to form a stem-loop structure, and such that a spacing distance between the first fluorescent group and the second fluorescent group is less than a limit spacing distance at which a fluorescence resonance energy transfer occurs;
in the condition that the solution to be tested comprises a large amount of the small molecule substance, the antibody specifically binds to the small molecule substance and loses binding to the small molecule substrate in the substrate protein conjugate, and the immune complex and the stem-loop structure are not formed.

3. The reagent combination for detecting the small molecule substance as claimed in claim 2, wherein the first fluorescent group is acridinium ester; the second fluorescent group is a quantum dot; the first nucleic acid molecule, the second nucleic acid molecule and the third nucleic acid molecule are single-stranded DNA molecules.

4. The reagent combination for detecting the small molecule substance as claimed in claim 3, wherein the quantum dot is a core-shell structure quantum dot, whose core layer material is selected from one or more of CdSe, CdS, CdTe, CdSeTe, CdZnS, ZnTe, CdSeS, PbS and PbTe, and whose shell material is selected from one or more of ZnS, ZnSe, ZnSeS, PbS and PbSeS; the quantum dot has a maximum absorption wavelength of 470 nm and a maximum emission wavelength of 605 nm; and the acridinium ester has a maximum emission wavelength of 430 nm.

5. The reagent combination for detecting the small molecule substance as claimed in any one of claims 1 to 4, wherein the first hybridization zone is ᵢₛₒGᵢₛₒCTᵢₛₒGAᵢₛₒGTT from a 5' end to a 3' end, and the sixth hybridization zone is AAᵢₛₒCTᵢₛₒCAᵢₛₒGᵢₛₒC from the 5' end to the 3' end;
a structural formula of ᵢₛₒG is: 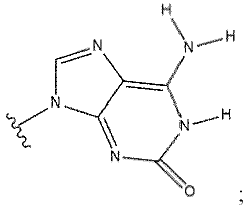 and
a structural formula of ᵢₛₒC is:

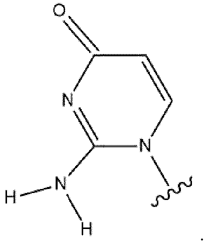

6. The reagent combination for detecting the small molecule substance as claimed in any one of claims 1 to 4, wherein the second hybridization zone is ᵢₛₒCAAᵢₛₒCᵢₛₒGAᵢₛₒC from a 5' end to a 3' end, and the third hybridization zone is ᵢₛₒGTᵢₛₒCᵢₛₒGTTᵢₛₒG from the 5' end to the 3' end;
a structural formula of ᵢₛₒG is: 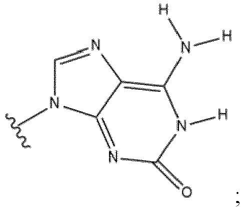 and
a structural formula of ᵢₛₒC is:

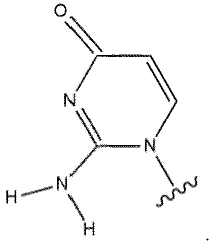

7. The reagent combination for detecting the small molecule substance as claimed in any one of claims 1 to 4, wherein the fourth hybridization zone is ᵢₛₒGᵢₛₒCTᵢₛₒGAᵢₛₒGAT from a 5' end to a 3' end, and the fifth hybridization zone is ATᵢₛₒCTᵢₛₒCAᵢₛₒGᵢₛₒC from the 5' end to the 3' end;
a structural formula of ᵢₛₒG is: 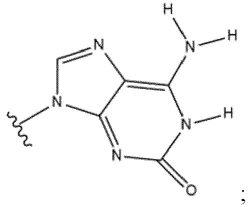 and
a structural formula of ᵢₛₒC is:

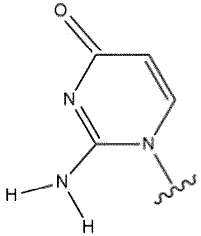

8. The reagent combination for detecting the small molecule substance as claimed in any one of claims 1 to 4, wherein a full-length sequence of the first nucleic acid molecule is a sequence formed by replacing at least a part or all of G by ᵢₛₒG and replacing at least a part or all of C by ᵢₛₒC in the sequence as shown by SEQ ID No:1;
a full-length sequence of the second nucleic acid molecule is a sequence formed by replacing at least a part or all of G by ᵢₛₒG and replacing at least a part or all of C by ᵢₛₒC in the sequence as shown by SEQ ID No:2;
a full-length sequence of the third nucleic acid molecule is a sequence formed by replacing at least a part or all of G by ᵢₛₒG and replacing at least a part or all of C by ᵢₛₒC in the sequence as shown by SEQ ID No:3;
a structural formula of ᵢₛₒG is: 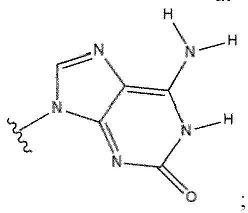 and
a structural formula of ᵢₛₒC is:

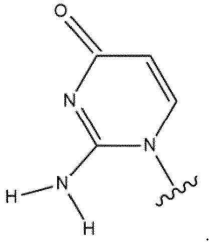

9. The reagent combination for detecting the small molecule substance as claimed in any one of claims 5 to 8, wherein,
a bonding manner of ᵢₛₒG and ᵢₛₒC is: 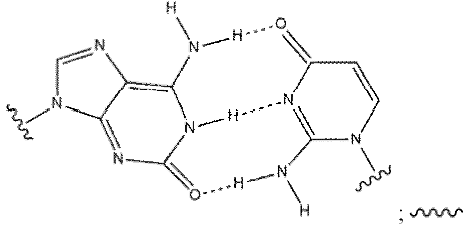 indicates a linkage with deoxyribose in a DNA molecule.

10. The reagent combination for detecting the small molecule substance as claimed in any one of claims 1 to 9, wherein the oxidation inhibiting reagent further comprises a carrier molecule, and a surface of the carrier molecule is bound to the antioxidant;
the antioxidant is selected from any one or more of cannabidiol, vitamin C, vitamin E, tea polyphenols, and glutathione; and
the oxidant comprises an alkaline solution of hydrogen peroxide.

11. The reagent combination for detecting the small molecule substance as claimed in claim 10, wherein the carrier molecule is graphene oxide; a carboxyl group on the graphene oxide binds to a hydroxyl group on the antioxidant via a sulfoxide oxide condensation agent, and the carboxyl group on the graphene oxide binds to an amino group on the antioxidant via 1-(3-dimethylaminopropyl)-3-ethylcarbondiimine hydrochloride.

12. A method for detecting a small molecule substance, comprising:
adding a first conjugate, a second conjugate, a third conjugate and an oxidation inhibiting reagent to a solution to be tested, and performing mixing to form a sample to be tested; wherein the first conjugate is formed at least by coupling a first nucleic acid molecule to an antibody, the antibody can specifically bind to the small molecule substance; the second conjugate is formed at least by sequentially coupling a substrate protein conjugate, a second nucleic acid molecule and a second fluorescent group, the substrate protein conjugate is formed at least by coupling the small molecule substrate to a scaffold protein, the small molecule substrate is the same as the small molecule substance; the third conjugate is formed at least by coupling a first fluorescent group and a third nucleic acid molecule; in the condition that the solution to be tested comprises or does not comprise a small amount of the small molecule substance, the antibody and the substrate protein conjugate form an immune complex, the first nucleic acid molecule, the second nucleic acid molecule and the third nucleic acid molecule form a stem-loop structure, and the first fluorescent group and the second fluorescent group are located on the same side of the stem-loop structure;
removing an oxidation inhibiting reagent for inhibiting the first fluorescent group from being oxidized from the sample to be tested, adding an oxidant for oxidizing the first fluorescent group to emit a first fluorescence, and collecting a second fluorescence according to a maximum emission wavelength of the second fluorescent group; and
obtaining a content of the small molecule substance according to an intensity of the second fluorescence and a one-to-one correspondence between the intensity of the fluorescence and the content of the small molecule substance in a case that the second fluorescence is collected.

13. The method for detecting the small molecule substance as claimed in claim 12, wherein a working concentration of the first conjugate in the sample to be tested ranges from 1 nM to 20 nM;
a working concentration of the second conjugate in the sample to be tested ranges from 1 nM to 20 nM;
a working concentration of the third conjugate in the sample to be tested ranges from 0.05nM to 0.2nM; and
a working concentration of the oxidation inhibiting reagent in the sample to be tested ranges from 15 µg/ml to 25 µg/ml.

14. The method for detecting the small molecule substance as claimed in claim 12, wherein the solution to be tested is derived from a whole blood sample, a serum sample or a plasma sample.

15. The method for detecting the small molecule substance as claimed in claim 12, wherein the small molecule substance comprises triiodothyronine, tetraiodothyronine, or progesterone.

16. A kit for detecting a small molecule substance, comprising:
a first storage tube storing at least a conjugate of a first nucleic acid molecule and an antibody, wherein the antibody is specifically capable of binding to the small molecule substance;
a second storage tube storing at least a conjugate of a substrate protein conjugate, a second nucleic acid molecule and a second fluorescent group; wherein the substrate protein conjugate is formed at least by coupling a small molecule substrate to a scaffold protein, the small molecule substrate is the same as the small molecule substance; the antibody and the substrate protein conjugate form an immune complex in the condition that the solution to be tested does not comprise or comprises a small amount of the small molecule substance;
a third storage tube storing at least a conjugate of a first fluorescent group and a third nucleic acid molecule; wherein the first nucleic acid molecule, the second nucleic acid molecule and the third nucleic acid molecule is capable of forming a stem-loop structure in the condition that the immune complex is formed, the first fluorescent group and the second fluorescent group are located on the same side of the stem-loop structure, a first fluorescence emitted by the first fluorescent group can excite the second fluorescent group to emit a second fluorescence, a content of the small molecule substance is obtained according to an intensity of the second fluorescence in the condition that the intensity of the second fluorescence is collected;
a fourth storage tube storing at least an oxidation inhibiting reagent capable of inhibiting the first fluorescent group from being oxidized; and
a fifth storage tube storing at least an oxidant capable of oxidizing the first fluorescent group to emit a first fluorescence.

17. The kit for detecting the small molecule substance as claimed in claim 16, wherein the first fluorescent group is acridinium ester; and the second fluorescent group is a quantum dot.

18. The kit for detecting the small molecule substance as claimed in claim 16, wherein the first nucleic acid molecule comprises a first hybridization zone and a second hybridization zone; the second nucleic acid molecule has a third hybridization zone and a fourth hybridization zone; the third nucleic acid molecule comprises a fifth hybridization zone and a sixth hybridization zone; the third hybridization zone is complementary to the second hybridization zone, the fifth hybridization zone is complementary to the fourth hybridization zone, and the sixth hybridization zone is complementary to the first hybridization zone;
at least a part or all of the first hybridization zone, the second hybridization zone, the third hybridization zone, the fourth hybridization zone, the fifth hybridization zone and the sixth hybridization zone comprise bases ᵢₛₒG and ᵢₛₒC;
a structural formula of ᵢₛₒG is: 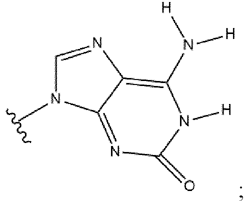 and
a structural formula of ᵢₛₒC is:

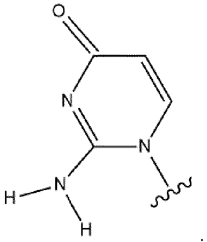

19. A system for detecting a small molecule substance, comprising:
a reaction vessel having an accommodating chamber capable of accommodating a solution to be tested;
a micro-injection pump communicated with the accommodation chamber through an injection pipeline, and injecting a mixture of a first conjugate, a second conjugate, a third conjugate and an oxidation inhibiting reagent into the accommodation chamber through the injection pipeline; wherein the first conjugate is formed at least by coupling a first nucleic acid molecule to an antibody, the second conjugate is formed at least by sequentially coupling a substrate protein conjugate, a second nucleic acid molecule and a second fluorescent group, the third conjugate is formed at least by coupling a first fluorescent group to a third nucleic acid molecule, and the oxidation inhibiting reagent comprises an antioxidant that inhibits the first fluorescent group from being oxidized to emit the first fluorescence;
an optical filter disposed on an exit light path of the first fluorescence to allow a second fluorescence emitted by the second fluorescent group to pass through;
an optical signal detection module disposed on the exit light path of the first fluorescence and located on a downstream side of the optical filter to acquire the second fluorescence transmitted from the optical filter;
a calculation module converting the second fluorescence into a digital signal, and obtaining a content of the small molecule substance in the solution to be tested according to a one-to-one correspondence between a fluorescence intensity and the content of the small molecule substance.
